(19) **Europäisches Patentamt / European Patent Office / Office européen des brevets**

(11) **EP 3 216 869 B1**

(12) **EUROPEAN PATENT SPECIFICATION**

(45) Date of publication and mention
of the grant of the patent:
**18.09.2019 Bulletin 2019/38**

(51) Int Cl.:
*C12N 15/113* (2010.01)   *A61K 31/7088* (2006.01)
*A61K 31/713* (2006.01)   *A61P 35/00* (2006.01)

(21) Application number: **16020075.4**

(22) Date of filing: **09.03.2016**

(54) **NUTRACEUTICAL PLANT DERIVED MICRORNA ELEMENTS FOR TREATMENT OF LEUKEMIA**

VON NUTRAZEUTISCHER PFLANZE ABGELEITETE MICRO-RNA-ELEMENTE ZUR BEHANDLUNG VON LEUKÄMIE

ÉLÉMENTS DE MICROARN DÉRIVÉS DE PLANTES NUTRACEUTIQUE POUR LE TRAITEMENT DE LA LEUCÉMIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(43) Date of publication of application:
**13.09.2017 Bulletin 2017/37**

(73) Proprietor: **Colizzi, Vittorio
00186 Roma (IT)**

(72) Inventors:
• **CIRILLO, Marco**
  Rome (IT)
• **GALGANI, Andrea**
  00189 Rome (IT)
• **DEL GALLO DI ROCCAGIOVINE, Flavia**
  00186 Rome (IT)
• **KENZO, Maurice**
  Dschang (CM)
• **MINUTOLO, Antonella**
  00139 Rome (IT)
• **MONTESANO, Carla**
  00169 Rome (IT)
• **MULEO, Rosario**
  56021 San Frediano a Settimo,
  Cascina (IT)
• **PIRRÒ, Stefano**
  00133 Rome (IT)
• **POTESTÀ, Marina**
  00133 Rome (IT)
• **MATIC, Ivana**
  00133 Rome (IT)

(56) References cited:
  WO-A1-2015/026249   WO-A1-2015/165535
  WO-A2-2008/073919   WO-A2-2008/154333
  WO-A2-2014/181344

• ANDREW R CHIN ET AL: "Cross-kingdom inhibition of breast cancer growth by plant miR159", CELL RESEARCH - XIBAO YANJIU, vol. 26, no. 2, 22 January 2016 (2016-01-22), pages 217-228, XP055300023, GB, CN ISSN: 1001-0602, DOI: 10.1038/cr.2016.13
• SIZOLWENKOSI MLOTSHWA ET AL: "A novel chemopreventive strategy based on therapeutic microRNAs produced in plants", CELL RESEARCH - XIBAO YANJIU, vol. 25, no. 4, 27 February 2015 (2015-02-27), pages 521-524, XP055300157, GB, CN ISSN: 1001-0602, DOI: 10.1038/cr.2015.25
• Vittorio Colizzi: "Nuovi integratori vegetali e Alimenti Funzionali: il caso della Moringa oleifera, pianta tropicale officinale", , 8 March 2014 (2014-03-08), pages 1-43, XP055300137, Retrieved from the Internet: URL:vglobale.it/public/files/2014/8Marzo-C olizzi-Moringa.pdf [retrieved on 2016-09-06]
• STEFANO PIRRÒ ET AL: "MicroRNA from Moringa oleifera: Identification by High Throughput Sequencing and Their Potential Contribution to Plant Medicinal Value", PLOS ONE, vol. 11, no. 3, 1 March 2016 (2016-03-01), page e0149495, XP055300028, DOI: 10.1371/journal.pone.0149495

**(Cont. next page)**

- **MARIA SALA-CIRTOG ET AL: "New insights of medicinal plant therapeutic activity-The miRNA transfer", BIOMEDICINE AND PHARMACOTHERAPY., vol. 74, 28 August 2015 (2015-08-28), pages 228-232, XP055300196, FR ISSN: 0753-3322, DOI: 10.1016/j.biopha.2015.08.016**

- **HONGWEI LIANG ET AL: "Regulation of mammalian gene expression by exogenous microRNAs", WILEY INTERDISCIPLINARY REVIEWS: RNA, vol. 3, no. 5, 27 June 2012 (2012-06-27), pages 733-742, XP055273729, United Kingdom ISSN: 1757-7004, DOI: 10.1002/wrna.1127**

**Description**

FIELD OF INVENTION

The present invention relates to the fields of molecular biology and medicine. The invention is based on the innovative concept of "cross-kingdom functional sequence homology" between plant and mammalian microRNAs (miRNAs), where plant miRNAs explicit a regulation mechanism into the host mammalian cell, comparable to the endogenous one. More specifically, the invention relates to identification of specific plant miRNA from *Olea europea, Moringa oleifera* and other Mediterranean and medicinal plants which inhibits *in vitro* proliferation of human acute leukemia cells, and is fit for the nutraceutical compositions comprising the miRNA.

STATE OF THE PRIOR ART

The subject of this invention is related to the use of synthetic *plant-miR-20* molecule and its plant-derived equivalent, for decreasing leukemia cell proliferation by regulating expression of cancer-related proteins. The goal of the present invention is to deliver a novel treatment of leukemia based on miRNA molecules from plants, which through the interaction with mammalian messenger RNA (mRNA) encoding oncoproteins, negatively regulate their expression and decrease *in vitro* proliferation of human leukemia cells.

MiRNAs are a class of small (17-25 nucleotides) non-coding RNAs that post-transcriptionally regulate gene expression by interacting with mRNAs. In the last years, miRNAs have been strongly associated with the regulation of critical biological events including development, differentiation, inflammation, apoptosis and carcinogenesis.

MiRNA-mediated gene regulation may occur via either mRNA degradation or preventing the mRNA from bending to a ribosome and being translated. Plant miRNAs need high complementarity to recognize their substrate and the target cleavage is considered the predominant pathway to repress gene expression. Several studies suggest that translational inhibition is also a common mechanism employed by plant miRNAs to repress gene expression. In animals, the semi-complementary interaction leads to a silencing achieved by translational repression [1-5]. In 2005, Brennecke and coworkers [6] reviewed for the first time the minimal requirements for a functional miRNA-mRNA duplex *in vivo*, introducing the concept of "functional sequence homology" related to common post-transcriptional regulations mediated by miRNAs. The study reported that any mismatch in positions 2 to 8 of the seed region strongly reduced the magnitude of the regulation of target. Moreover, the minimal number of complementary nucleotides in 5' sequence necessary to confer target regulation is 4 or 5.

In humans, several reports have associated the expression profiles of specific miRNAs with certain physiological and pathological stages, tumourigenesis or even patient response to treatment. Studies have shown that changes in the expression levels of numerous miRNAs are associated with various cancers [7-9]. MiRNAs have also been implicated in regulating cell growth and cell and tissue differentiation - cellular processes that are associated with the development of cancer. Thus, in medicine miRNAs became new diagnostic and prognostic biomarkers [10, 11] and have been incorporated in a few novel therapies for treating several human disorders [12, 13].

MicroRNA-34 (miR-34) is a potent tumour suppressor that shows a loss of function in many solid and hematological cancer types [14-16]. It inhibits a broad range of cancer cells, presumably by repressing a plethora of oncogenes that control proliferation, senescence, apoptosis and metastasis [17]. MiR-34 can also interfere with the growth of cancer stem cells [18, 19], providing a strong rationale for the development of a miR-34 therapy. Evidence for the therapeutic application of miR-34 has been generated in murine tumour models of lung, liver, prostate and lymphoma that showed robust tumour inhibition in response to the systemic delivery of nanoparticles loaded with synthetic miR-34 mimics [20-23]. A miR-34-based therapy is currently in Phase I clinical trials.

The miR-34 family consists of three members: miR-34a, miR-34b, and miR-34c and their expression is induced by p53 as a result of cell damage, oxidative stress and DNA damage [24-26]. The members of the miR-34 family repress the post-transcriptional expression of genes that are involved in cell cycle control and to DNA damage such as, for example, Bcl-2, Sirt1, c-Myc. The blocking of the expression of these genes on a post-transcriptional level due to miR-34 action, induces apoptosis, cell cycle arrest in G1, senescence and inhibition of migration [27]. Different bioinformatic and *in vitro* studies indicated that miR-34a regulates a variety of target mRNAs, including cyclin dependent kinase 4/6 (CDK4/6), E2F transcription factor3 (E2F3), Cyclin E2, B cell lymphoma 2 (Bcl-2) and NAD dependent deacetylase sirtuin 1 (Sirt1) [28, 29]. Bcl-2 family members were originally characterized with respect to their roles in regulating apoptosis through complex interactions that dictate the integrity of the outer mitochondrial membrane. As an NAD induced deacetylase, SIRT1 is a transcriptional regulator and can inhibit the expression of pro apoptotic proteins [30]. SIRT1 regulates p53 dependent apoptosis by deacetylation and destabilizing p53. It has been confirmed that SIRT1 mediates miR-34a induced apoptosis by regulating p53 activity. A positive feedback loop has been identified, in which p53 induces expression of miR-34a, suppressing SIRT1 and increasing p53 activity [31].

Patent application US 20090227533 A1 (publ. 10 Sep, 2009) concerns methods and compositions for identifying genes or genetic pathways modulated by miR-34, subsequently using miR-34 to modulate a gene or gene pathway for the treatment of diseases or conditions that are affected by miR-34 miRNAs. In the patent application WO 2008137862 A2 (publ. 13 Nov 13, 2008) methods of using mir-34 as a biomarker for the estimation of p53 functional status in cells are described. The invention relates to use of miR-34 and small interfering RNAs (siRNAs) functionally and structurally related to miR-34 for the treatment of cancer. Patent application WO 2014209970 A1 (publ. 31 Dec, 2014) is based on the discovery that miR-34 is independent of p53 and has a more central role during tumor suppression that is uncoupled from p53. The invention concerns screening the subject for the presence or absence of at least one prerequisite biomarker for miR-34 activity and administering a miR-34 therapeutic to the subject if the prerequisite biomarker(s) for miR-34 activity is determined to be present or absent. Patent application WO 2009058766 A2 (publ.7 May, 2009) relates to methods for diagnosing, treating, and monitoring subjects with HPV-infected and/or cancer cells, particularly methods of reducing the tumor growth by increasing the amount of a miR-34a nucleic acid in cancer cells. The increase is reached through the internalization of miR-34a nucleic acid into the cells for the subsequent targeting of cell cycle regulatory genes and restoring apoptosis in cancer. Patent application US 8586727 B2 (publ. 19 Nov, 2013) concerns methods and compositions involving miR-34 mimics, including miR-34 and miR-34c mimics, such as double-stranded RNA molecules (dsRNA) with modified nucleotides having an active strand with a miR-34a or miR-34c sequence and a complementary passenger strand. Patent application US 8933051 B2 (publ. 13 Jan, 2015) is based on the observation that miR-34a shows strong antiproliferative effects when overexpressed in diffuse large B-cell lymphoma (gDLBCL) cell lines, or when delivered intratumourally or systemically in xenograft models of DLBCL. The invention relates to microRNA-34a for use in the preparation of a medicament for the treatment of B-cell lymphoma, and for a method of treatment of B-cell lymphoma comprising administering miR-34a.

Plant miRNAs are known to function in a sequence-specific manner to silence specific protein-coding genes at the post-transcriptional level by targeting the 3' untranslated region (3'UTR) of mRNA [1]. In plants, miRNAs repress gene expression by pairing with near-perfect complementary sequences primarily in the mRNA coding region to guide cleavage and translational repression [32]. They act as key regulators in several processes, such as development, stress response and other physiological processes [33, 34].

In 2012, Zhang L. et al demonstrated that miRNA168a and other exogenous miRNAs abundant in rice plants are present in the sera and tissues of mice acquired orally, through food intake [35]. Functional studies *in vitro* and *in vivo* demonstrated that miR-168a binds the human/mouse low-density lipoprotein receptor adapter protein 1 (LDLRAP1) mRNA, inhibits its protein expression in liver and decreases the LDL removal from mouse plasma. For the first time, Zhang and co-workers demonstrated that microRNAs contained in vegetal foods regulate mRNA translation in a fashion of mammalian functional miRNA. Despite controversial opinions [36, 37], these findings have opened the extraordinary field of cross-kingdom regulation. More recently, functional miRNA studies demonstrated that ICR mice fed with plant total RNAs in quantities of 10-50 μg, showed miR-172 from *B. olearacea* have most abundant exogenous microRNA in the sera, feces and tissues as intestine, stomach, spleen, liver and kidney [38]. Recently, Chin et al. [39] reported that plant miR-159 abundance in the serum was inversely correlated with breast cancer incidence and progression in patients. Only in breast cancer cells, and not in non-cancerous mammary epithelial cells, a synthetic mimic of miR159 was capable of inhibiting proliferation by targeting TCF7 that encodes a Wnt signaling transcription factor, leading to a decrease in MYC protein levels. Oral administration of miR159 mimic significantly suppressed the growth of xenograft breast tumours in mice. These recent reports on the cross-kingdom regulation by plant miRNAs open new opportunities for natural medicine and the treatment of many known diseases.

Patent application WO 2014181344 A2 (publ. 13 Nov, 2014) concerns bioactive apolar extract of plant material containing genetic material (miRNA) along with NADPH Oxidase inhibitors from plants for a therapeutic effect in controlling and treating infectious diseases, tumors, cancers, hepatic diseases and hyperlipidemia. In the patent application WO 2015026249 A1 (publ. 26 Feb, 2015), a use of plant-derived miR-172 molecule or its synthetic equivalent, selected from amongst miR-172a or miR-172b, for decreasing inflammatory processes in the organism is described. Further, the invention relates to a method of decreasing B and T lymphocyte proliferation, as well as a method of reducing FAN (Factor Associated with Neutral Sphingomyelinase Activation) protein level with the goal to deliver a novel therapeutic method based on miRNA molecules, which interact with mRNA encoding FAN protein, negatively regulate its expression and decrease the inflammatory response of the organism. Patent application 20150291962 (publ. 15 Oct, 2015) relates to a use of a micro RNA molecule miR-2911 or an extract containing miR-2911 for preparing a pharmaceutical composition for treating viral cold. The invention is based on method for in vitro non-therapeutic regulation of the expression of non-plant target genes such as bacterial gene, viral gene, chlamydial gene, yeast gene, and animal gene.

Several miRNA discovery methods have been employed by researchers, such as computational prediction and others [40]. These techniques are not effective without sequence information. Initially, miRNAs were identified by the traditional Sanger sequencing of relatively small-size cDNA libraries of plant small RNAs (sRNAs) from *Arabidopsis*, rice and poplar (*Populus* spp.) [41]. Comparison of miRNAs from these species led to the conclusion that plant miRNAs are conserved within and between different plant species. However, there are some miRNAs not present in the genomes of different species, suggesting that they have evolved more recently [42]. High-throughput sequencing technologies have contributed to markedly expanding the knowledge of the sRNA universe in the eukaryotic cell by bringing into scene a number of newly evolved and species-specific miRNAs without any previous sequence information [43].

During last years, several computational approaches have been elaborated to predict miRNA-mRNA interactions: miRanda [44], TargetScan [45], PITA [46], PicTar [47]. Most algorithms use experimental knowledge to develop a specific scoring system, such as miRNA-mRNA partial complementarity, seed region, target position, sequence conservation features, etc. In 2013, Coronnello and Benos [48] developed a web tool for combinatorial miRNA target prediction, called ComiR that combines four popular scoring schemes (miRanda, PITA, TargetScan and mirSVR) to compute the potential of a gene to be targeted by a set of miRNAs. Recently, an algorithm has been designed, implemented and tested to predict the action of plant miRNAs inside the mammalian host cell environment, leading up to cross-kingdom interactions by comparing plants and mammalians miRNAs, searching for functional sequence homologies between them via web-software called MirCompare [49]. Moreover, this algorithm enables investigation of potential tumor suppressing miRNAs in plants with reported anti-tumor activity such as *Olea europea* and *Moringa oleifera* plants. As study-case, authors provided *O. europaea* and *H. sapiens* mirnomes to MirCompare, in order to select a strict number of plant miRNAs member with a potential genomic regulative role into human cells.

*O. europaea* is one of the most economically important fruit crops in the world. The olive oil has been declared as a healthy medicine for cardiovascular protection (qualified health claim) by the Food and Drug Administration (www.fda.gov), due to its protective effect against cardiovascular diseases, as resulting from label approved for a conventional food. Patent application US 20090048187 A1 (publ. 19 Feb, 2009) relates to a composition and method for preventing and treating cancer and modulating cell proliferation using compositions extracted from pinoresinol-rich *O. europaea* plant by contacting one or more cells with a pharmaceutical effective amount of a polyphenolic composition isolated from *O. europaea* olives. In the patent application WO 2013162484 A1 (publ. 31 Oct, 2013) a pharmaceutical product produced from the olives (*O. europaea*), in combination with figs (*Ficus carica*) is used as a supplement and supportive in eliminating formed cancer cells and in preventing the formation of cancer cells.

*Moringa oleifera* Lam, a naturalized species from the monogeneric family *Moringaceae*, is one of the best known, most widely distributed and most useful nutritional and medicinal plants [50-52]. Several parts of the *Moringa* tree are edible (e.g., pods, seeds, flowers and leaves) and are used in many countries (including many parts of Africa) for their high nutritional value [51, 52]. Almost all parts of this plant can be used in the treatment of inflammation or infectious diseases along with cardiovascular, gastrointestinal, haematological and neoplastic diseases [52, 53]. In 2014, Jung IL reported a tumor suppressor activity in a soluble extract of *M. oleifera* leaves [54]. The author found an abnormal ribosomal RNA (rRNA) pattern and down-regulation of many genes and proteins involved in cell transformation and proliferation in mammalian cells treated with this extract. He concluded that the cold water-soluble extract of *M. oleifera* induced rRNA degradation. In the patent application US 20060222682 A1 (publ. 5 Oct, 2006) beneficial properties of *Moringa* plant are described. The leaves, seeds, and fruit of the *Moringa* plant are used for production of a biologically metabolized nutritional composition for health, well-being, and for treatment of ailments. Patent application CN 104224862 A (publ. 24 Dec, 2014) relates to a composition containing a *M. oleifera* leave water soluble extract as an effective component for treating or preventing cancer cell proliferation, more precisely A549 lung cancer cells.

Previous reports related to the use of *Olea* and *Moringa* in the treatment of cancer and cancer related diseases have not so far neither associated their medicinal properties with the presence of miRNA molecules nor provided methods of utilizing said molecules for therapy of leukemia. For purposes of the present invention, authors herein provide the first report of linking putative *O. europaea* miRNAs identified based on bioinformatics analysis, with a cross-species control of human gene expression. The widespread cultivation and consumption of *Olea* and *Moringa* for nutritional and medicinal purposes, brings humans into close contact with products and extracts of these plant species. The potential for miRNA transfer should be evaluated as one possible mechanism of action to account for beneficial properties of these valuable plants. Given that: food-derived miRNAs are stable to cooking considering their short sequences, are resistant to enzymatic digest in the gastrointestinal tract, are stable in animal serum, and arc able to regulate gene expression in the organisms that ingest them [55], a nutraceutical composition containing optimal concentration of active leukemia cells proliferation suppressing plant putative miRNAs could deliver a natural non-toxic therapy approach, advantageous in respect to currently available chemotherapy agents which often render concurrent toxic effects both to the body and the cancer. Realization of the goal so specified, as well as showing of proofs that specific miRNA molecules are responsible for certain medical properties of some plants, have been achieved in the present invention.

SUMMARY OF THE INVENTION

The present invention was made in view of the prior art described above, and the goal of the present invention is to provide a novel advantageous non toxic method of treating leukemia by means of using plant derived *plant-miR-20* molecule.

The disclosure refers to identification of putative plant miRNA with functional homologies with mammalian one by using a novel algorithm for the software MirCompare, that enables for the comparison between plants and mammalians miRNAs and their highly stringent selection. MirCompare analysis on *O. europaea* plant highlights plant miRNA with a putative role in human cross-kingdom interaction, particularly in the endogenous regulation machinery controlling tumour related genes. More specifically, transfection experiments evidenced that *plant-miR-20* identified by the bioinformatics inhibits the translation of SIRT1 and Bcl-2, when its synthetic mimics are transfected into Jurkat cells (derived from human immortalized T lymphocytes) and THP1 cells (derived from human immortalized monocytes). The present disclosure is directed to a nutraceutical composition for treating leukemia, comprised of a designated formulated combination containing active plant miRNA.

Therefore a first object of the disclosure is the identification of functional sequence homologies between plant derived (*plant-miR-20)* and mammalian miRNA (miR-34) and subsequent identification of the target human mRNAs and related oncoproteins (SIRT1 and Bcl-2).

A second object of the disclosure is evaluation of the *in vitro* inhibition of cell proliferation, of apoptosis and killing of human leukemia cells by putative plant miRNA.

A third object of the disclosure is the identification in plant tissues, of putative miRNA homologues.

A fourth object of the disclosure is evaluation of the *in vitro* inhibition of cell proliferation, of apoptosis and killing of human cancer cells by plant extracts containing optimal amount of said putative miRNA.

Further advantages involve preparation of different plant extracts containing said miRNA and clinical utilization of foods (eg. prepared pasta and soup) with miRNA from plant tissues as nutraceutical aliments for leukemia patients.

DETAILED DESCRIPTION OF THE FIGURES

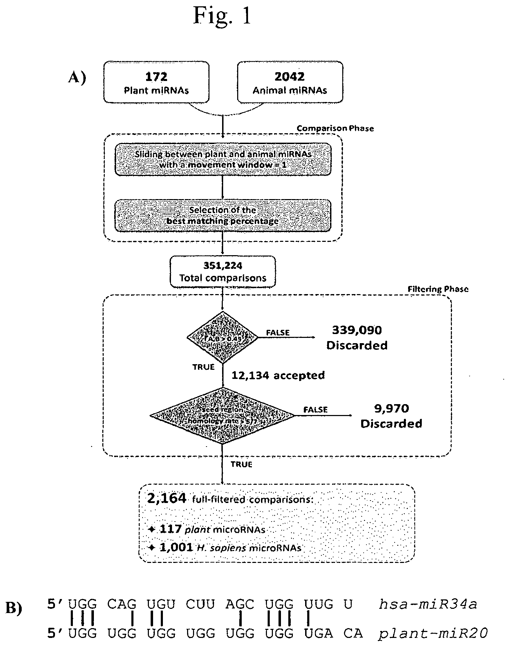

Figure 1 is a schematic workflow of the MirCompare algorithm in which *O. europaea* and *H. sapiens* mirnomes are provided, in order to select a strict number of plant miRNA members with a potential genomic regulative role into human cells. In the comparison phase, the best alignment rate between each couple of miRNAs is evaluated. In the filtering phase, functional homologies are selected using a double-step approach based on the evaluation of the overall homology and the seed region stringency: 117 plant miRNAs and 1001 human miRNAs comparisons and the alignment/homology sequences percentage between *hsa-miR-34a* and *plant-miR-20* are evaluated.

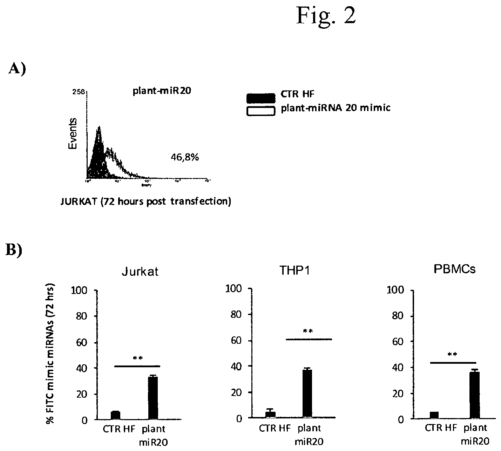

Figure 2 reports the transfection efficiency of Jurkat and THP-1 cell lines and PBMCs (Peripheral Blood Mononuclear Cells) with the three fluorescein isothiocyanate (FITC) labeled *plant-miR-20* mimic assessed by monitoring percentage of fluorescent positive (FL1- positive) cells by flow cytometry analysis.

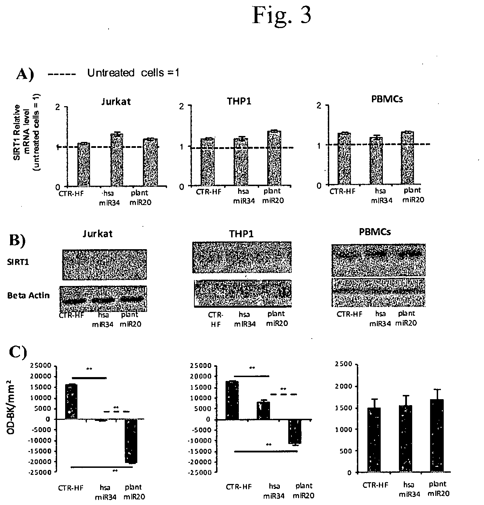

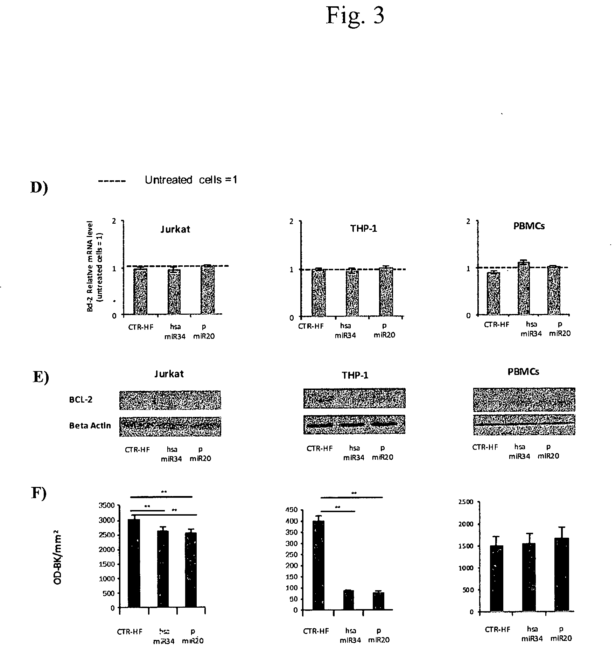

Figure 3 reports analysis of the mRNA and protein expression of SIRT1 and Bcl-2, specific targets of *hsa-miR-34a* regulation, upon transfection with *plant-miR-20* mimic in Jurkat and THP-1 cell lines compared to negative PBMC control.

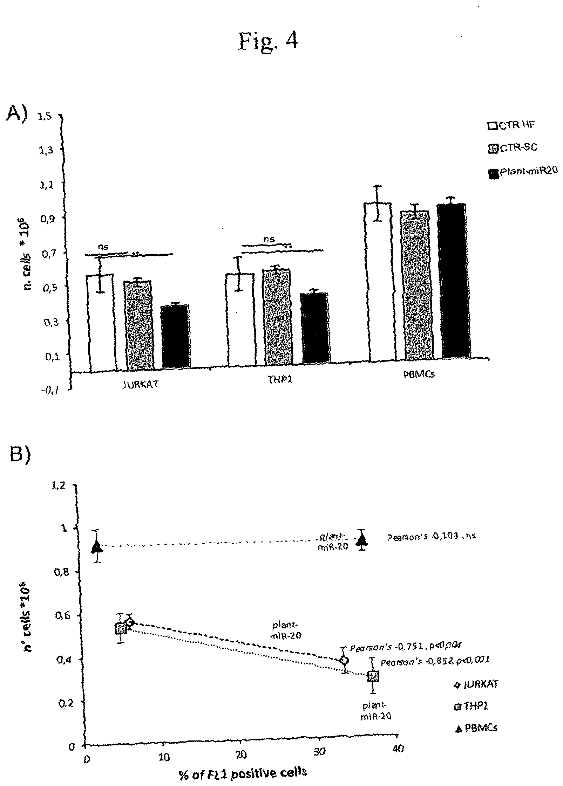

Figure 4 reports effect of *plant-miR-20* and *hsa-miR-34a* mimics transfection on cell viability and apoptosis in Jurkat and THP-1 cell lines compared to negative PBMC control analysed by flow cytometry and trypan blue assay.

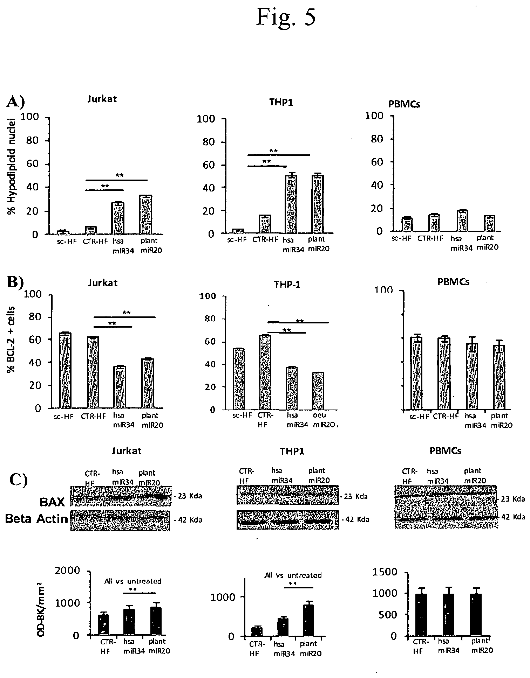

Figure 5 reports analysis of apoptosis upon transfection with *plant-miR-20* and *hsa-miR-34a* mimics in THP-1 and Jurkat cells compared to negative PBMC control by assessment of percentage of hypodiploid nuclei, expression of the intracellular anti-apoptotic protein Bcl-2 and Western Blot analysis for the expression of BAX protein involved in intrinsic signalling pathways of programmed cell death.

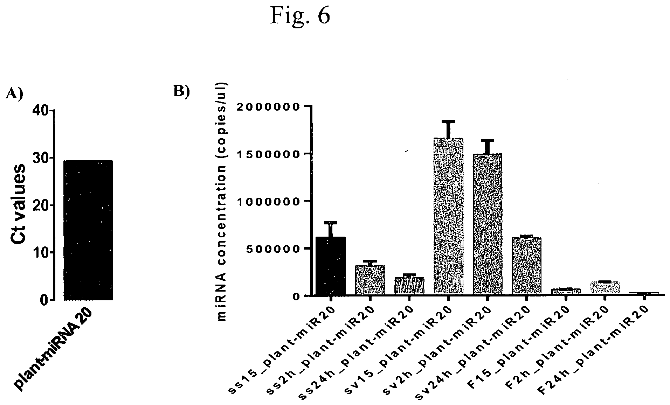

Figure 6 is a graphic representation of quantitative analysis of homologous *plant-miR-20* molecule in seed (panel A) and soluble *M. oleifera* extracts from different tissues (leaf, F; immature seed, SV; dried mature seed, SS) (panel B). Data are expressed as Ct values and absolute concentrations (miRNA copies per microliter) from qRT-PCR (quantitative Real Time Polymerase Chain Reaction) analysis.

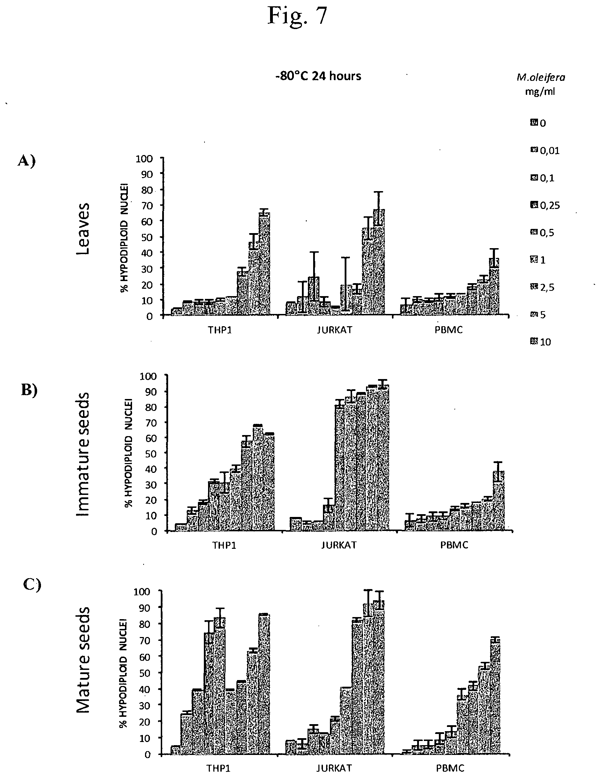

Figure 7 reports analysis of apoptosis in THP-1 and Jurkat cells compared to negative PBMC control by percentage evaluation of hypodiploid nuclei. All cells were treated for 72h with different concentrations (from 0 to 10 mg/ml) of leaf (F) extracts (panel A), immature seed (SV) and dried mature seed (SS) extracts of *M. oleifera* previously prepared by freezing (-80°C).

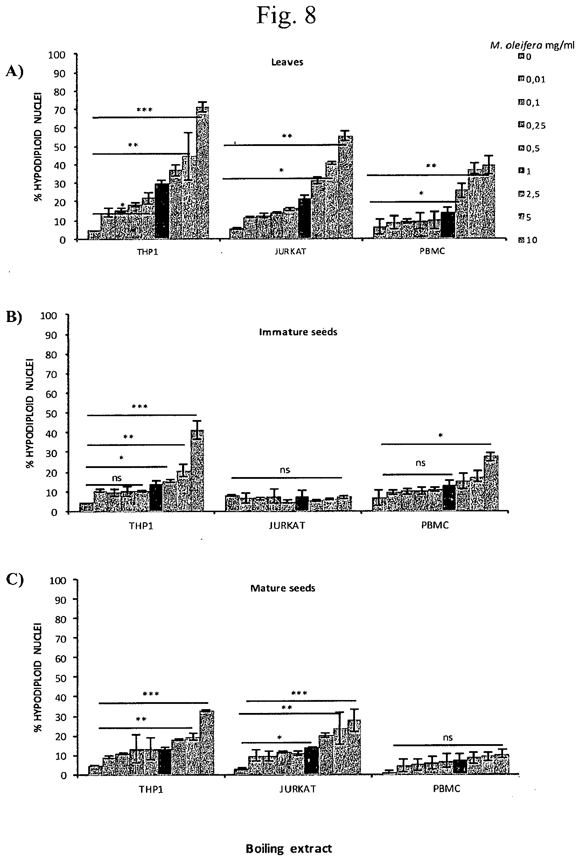

Figure 8 reports analysis of apoptosis in THP-1 and Jurkat cells compared to negative PBMC control by percentage evaluation of hypodiploid nuclei. All cells were treated for 72h with different concentrations (from 0 to 10 mg/ml) of leaf (F) extracts (panel A), immature seed (SV) and dried mature seed (SS) extracts of *M. oleifera* previously prepared by boiling (100°C).

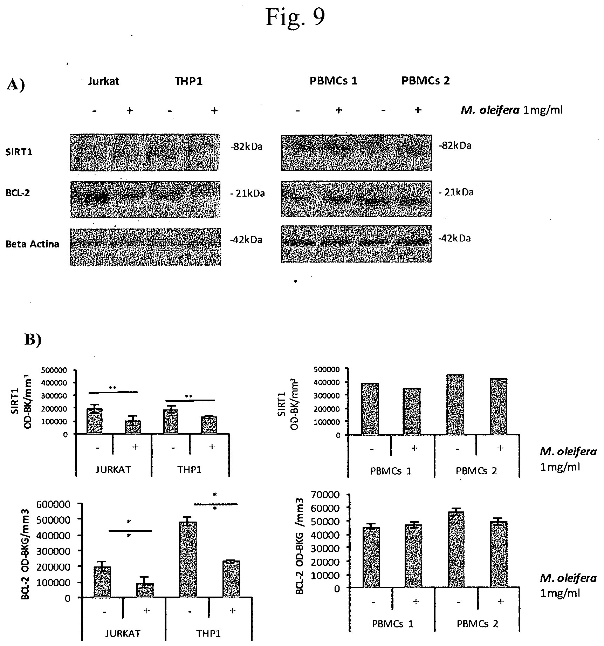

Figure 9 reports analysis of protein expression of SIRT1 and Bcl-2 (panel A), specific targets of *hsa-miR-34a* regulation, in Jurkat and THP-1 cell lines, compared to PBMCs from two healthy donors, upon treatment with 1 mg/ml of boiled *M. oleifera* dried mature seed (SS) extract (panels A and B). In densitometric analysis, values are expressed as OD-Bkg/mm2 (means±SD of three independent experiments) and the susceptibility to apoptosis of THP1, Jurkat and PBMCs upon treatment with 1 mg/ml of boiled *M. oleifera* mature seed (SS) extract.

DETAILED DESCRIPTION

The present disclosure is comprised of the following aspects, described in detail below.

Based on the strong evidence that plant miRNAs inside mammalian cells use the endogenous machinery for post-transcriptional regulation of target genes [35] the authors have conceived an innovative model in which a plant and mammalian miRNAs might share the same target genes if they show:

1) a sequence homology rate higher than a specific percentage,

2) a strict sequence homology, related to the seed-region.

According to this model, a MirCompare algorithm able to compare libraries of miRNAs belonging to organisms from plant and animal kingdoms is developed, in order to find cross-kingdom functional homologies. In a preferred embodiment the MirCompare workflow starts with the upload of two different FASTA files, containing miRNA sequences from different animal and plant organisms. These sequences are obtained from specialized databases: miRBase, DIANA TarBase, PMRD, etc. or high-throughoutput sequencing (miRNA seq). The computational process is split into two main phases: comparison and filtering.

In the first phase (comparison), in order to evaluate the best alignment rate between each couple of miRNAs (A and B), the shorter sequence is sliding on the longer one, using a sliding window of 1 nucleotide and for each step an alignment score $S_{A,B}$ is calculated.

$$S_{A,B} = matches_{A,B} \Big/ \max(length(A), length(B))$$

When all the possible alignment rates for each couple of uploaded miRNAs have been generated, the algorithm selects the best comparison rate (r):

$$r_{A,B} = \max_i(S_{A,B})$$

where

$$0 \leq i \leq \max(length(A), length(B)) - 1$$

The comparison phase computes a total of N x M different comparison rates, where N and M are respectively the length of the first and second miRNAs datasets.

In the second phase (filtering), the rules for the cross-kingdom functional sequence homology are applied. This procedure is divided in two consequential parts:

a) filtering: based on the overall sequence homology rate;

b) selection: related to the seed-region sequence homology.

In the part a), in order to identify a cut-off for the best comparison rate between each miRNA couple ($r_{A,B}$), $10^4$ and $10^6$ stochastic comparisons are generated, using two scramble sets of $10^2$ and $10^3$ sequences, respectively. According to Wang et al. [56], base composition of mammalian miRNAs is GU-rich and shows an evolutionary conservation. For this reason, the sets of random sequences according to the experimental evidences are built. In particular, each set is composed by sequences of length between 17 and 25 nucleotides with a percentage of A and C of 20% and G and U of 30%. In order to establish the minimum r-value threshold able to produce a statistically significant alignment between a couple of miRNA sequences, the r-value frequency distribution in above described stochastic comparison analysis is plotted. For each generated r-value, a p-value is calculated:

$$p\text{-}value = \frac{\sum (num\ occurrences(r_{A,B}) | r_{A,B} \geq threshold)}{\sum (num\ occurrences(r_{A,B}))}$$

In both the casual comparisons, a peak on 0 is observed, confirming the casualty of the alignment. According to the **r**-value distributions, similar statistically significant cut-offs are obtained. In the case of $10^4$ comparisons, the lowest threshold value that generates a p-value $\leq 0.05$, is 0.48, while in the case of $10^6$ is 0.46. For this reason, the **r**-value threshold is fixed to 0.48.

In the part b) the magnitude of target regulation is not affected by a mismatch at positions 1, 9, or 10 but any mismatch from positions 2 to 8 causes a strong reduction. Additionally, a minimal complementarity of 4 or 5 nucleotides into the seed region is necessary to confer target regulation. Therefore, an additional filter is applied, selecting only the comparisons that show a homology rate value higher than 5 up to 7 nucleotides, into the seed region.

In order to predict all possible cross-kingdom targets in human, it is assumed that plant microRNAs regulate host mRNA translation as well as mammalian miRNAs. As input dataset in a double-step analysis, the MirCompare predicted human miRNAs are used. The first step of the procedure is extraction of a ranked list of genes that might be regulated by submitted miRNAs by using COMIR [47]. The COMIR output is provided as a list of couples "target gene - miRNA" to subsequent step. Diana TarBase [57] is used to select only the COMIR gene-miRNA interactions output that have already been experimentally validated in literature. The synergic use of a computational and experimental target prediction approaches allows extracting two lists: the first one with cross-validated miRNA-mRNA interactions and another one containing only computational predicted ones that will be validated in future experiments.

In an embodiment the method comprises of a further step in which miRNA-mRNA Free Energy is calculated. For the calculation of the miRNA-mRNA free energy variations a custom script that uses the miRanda algorithm [44] to calculate the free energy of duplex formation has been developed. For each plant miRNA sequence, the target mRNAs sequences are provided in FASTA format. After the generation of the output file, the script also extracts the best free energy value for each target, providing also the pairing region. The settings provided to miRanda are as follow: pairing score > 150, energy score < -7, forcing to search for a strict pairing region (no gaps for the miRNA seed region).

In a preferred extract, the seeds from *M. oleifera* trees, grown in the District of Dschang in West Cameroon in a biological agriculture (without use of chemical fertilizer) by the Cooperative of Medical Plant Producers SOCOPOMO are collected at pods maturity before they split open and fall to the ground. They are stored at -80°C for further use. Germination is performed in a greenhouse by placing the seeds on paper soaked in sterile water. Total RNA is isolated from defined plant tissue by using mirVana kit (Ambion, USA), according to the manufacturer's protocol. RNA quality and quantity are evaluated by Agilent 2100 Bioanalyzer (Agilent Technologies, Waldbroon, Germany) and by Spectrophotometer (SmartSpec Plus, Bio-Rad, USA). The expression level of different miRNAs is validated using poly(A)-tailed qRT-PCR method. According to the manufacturer's protocols (Exiqon A/S, Vedbaek, Denmark), a poly-A tail is added to the mature miRNA templates (20ng). cDNA is synthesized using a poly-T primer with a 3' degenerate anchor and a 5' universal tag at 42°C for 60 min followed by heat-inactivated for 5 min at 95°C. To provide a control for the quality of the cDNA synthesis reaction and the PCR, RNA spike-in (UniSp6) is added to the sample prior to cDNA synthesis. The cDNA template is then amplified using miRNA-specific and LNA™-enhanced forward and reverse primers. SYBR®Green is used for detection. The reactions are carried out in an Rotor-Gene®Q 72-Well Rotor (Qiagen, USA) with the following amplification conditions: activation/denaturation at 95°C for 10 min followed by 40 cycles of denaturation at 95°C for 10s, annealing and extension together at 60°C for 60 sec. Finally, melting analysis is performed to confirm the absence of false-positive peaks. Two controls (no template control and no Reverse Transcription control) are included in all reactions.

In another preferred example, said plant includes medicinal plants, fruit and vegetable plants, and ornamental plants; preferably includes *Abies alba, Abutilon theophrasti, Acanthus mollis, Acer campestre, Achillea spp, Aconitum napellus, Acorus calamus, Actaea spicata, Adiantum capillus veneris, Aesculus hippocastanum, Agave americana, Agrimonia eupatoria, Agropyrum repens, Agrostemma githago, Ailanthus altissima, Ajuga reptans, Alchemilla vulgaris, Alchemmila alpina, Alkanna tinctoria, Alliaria petiolata, Allium cepa, Allium porrum, Allium sativum L., Aloe spp, Althaea officinalis, Amaranthus retroflexus, Ambrosia artemisiifolia, Ammi majus, Amorphophallus konjac, Anacardium occidentaliss, Anagallis arvensis, Ananas sativus, Anchusa officinalis, Anemone nemorosa, Anethum graveolens, Angelica archangelica, Angelica sylvestris, Annona muricata, Antennaria dioica, Anthriscus cerefolium, Aquilegia vulgaris, Arbutus unedo, Arctium lappa L, Arctostaphylos uva-ursi, Aristolochia clematitis, Aristotelia chilensis, Arnica montan, Artemisia absinthium, Artemisia dranunculus, Artemisia vulgaris, Asarum europaeum, Asparagus officinalis, Asperula odorata, Aster amellus, Atriplex hortensis, Aucuba Japonica, Ballota nigra, Balsamita major, Barbarea vulgaris, Bellis perennis, Berberis vulgaris, Betula pendula, Bixa orellana, Borago officinalis, Boswellia serrata, Bryonia dioica, Buddleja davidii, Buxus sempervirens, Calamintha nepeta, Calendula arvensis, Calendula officinalis, Calluna vulgaris, Caltha palustris, Camellia sinensis, Campanula rapunculus, Cananga odorata, Cannabis sativa, Capparis spinosa, Capsella bursa pastoris, Capsicum spp, Cardamine bulbifera, Cardamine hirsuta, Carlina acaulis, Carthamus tinctorius, Carum carvi, Castanea sativa, Cedrus atlantica, Centaurea cyanus, Centaurium erythraea, Centella asiatica, Ceratonia siliqua, Cercis siliquastrum, Cetraria islandica, Chamomilla matricaria, Chelidonium majus, Chenopodium album, Chrysanthemum cinerariaefolium, Cichorium intybus, Cinchona officinalis, Cirsium arvense, Cirsium vulgare, Cistus monspeliensis, Citrus limon, Clematis vitalba, Clerodendrum trichotomum, Clinopodium vulgare, Cnicus benedictus, Cochlearia officinalis,*

*Cola acuminate, Conium maculatum, Convolvulus arvensis, Coriandrum sativum, Corylus avellana, Crataegus monogyna, Crepis vesicaria, Crocus biflorus, Crocus sativus, Croton lechleris, Cruciata laevipes, Cucurbita pepo, Cyclanthera pedata, Cymbalaria muralis, Cymbopogon spp, Cynara scolymus, Cynodon dactylon, Cytisus scoparius, Datura stramonium, Daucus carota, Descurainia sophia, Dictamnus albus, Diplotaxis tenuifolia, Dipsacus fullonum, Drosera rotundifoli, Dryopteris felix-mas, Duchesnea indica, Echinacea pallida, Echinacea purpurea. Echinops ritro, Echium vulgare, Elutherococcus senticosus, Epilobium hirsutum, Equisetum arvense, Erica carnea, Erigeron canadensis, Eriobotrya japonica, Erodium cicutarium, Eruca sativa, Elyngium campestre, Eschscholtzia californica, Eucalyptus globulus, Eugenia caryophyllata, Eupatorium cannabinum, Euphorbia lathyris, Euterpe oleracea, Fagus sylvatica, Ficus carica, Filipendula ulmaria, Foeniculum vulgare Miller subsp. vulgare var. dulce, Fragola selvatica, Frangula alnus, Fraxinus excelsior, Fucus vesiculosus, Fumaria officinalis, Galega officinalis, Galeopsis pubescens, Galeopsis tetrahit, Galinsoga parviflora, Galium aparine, Galium verum, Garcinia cambogia, Gentiana lutea, Geranium dissectum, Geranium molle, Geum urbanum, Ginkgo biloba, Glechoma hederacea, Globularia punctata, Glycyrrhiza glabra, Gratiola officinalis, Grindelia robusta, Gymnema sylvestris, Hamamelis virginiana, Harpagophytum procumbens, Hedera helix, Helianthus annuus, Helianthus tuberosus, Helichrysum italicum, Herniaria glabra, Hibiscus syriacus, Hieracium pilosella, Hippophae rhamnoides, Humulus lupulus, Hydrangea macrophylla, Hyoscyamus niger, Hypericum perforatum, Hypochoeris radicata, Hyssopus officinalis, Ilex aquifolium, Illicium verum, Impatiens glandulifera, Impatiens noli-tangere, Inula helenium, Iris psezidacorus, Isatis tinctoria, Jacobaea vulgaris, Jasminum officinale, Juglans regia, Juniperus communis, Juniperus oxycedrus, Lactuca serriola, Lamium album, Lamium amplexicaule, Lamium galeobdolon, Lamium maculatum, Lamium purpureum, Lapsana communis, Laurus nobilis, Lavandula spp, Leontopodium alpinum, Leonurus cardiac, Lepidium virginicum, Leucanthemum vulgare, Levisticum officinale, Liatris spicata, Linaria vulgaris, Linum usitatissimum, Lippia citriodora, Lithospermum officinale, Lobularia maritime, Lonicera caprifolium, Lonicera japonica, Lotus corniculatus, Lunaria annua, Lycium barbarum, Lycopus europaeus, Lysimachia vulgaris, Lythrum salicaria, Mahonia aquifolium, Malus domestica, Malva alcea, Malva silvestris, Matricaria chamomilla, Medicago sativa, Melilotus officinalis, Melissa officinalis, Melittis melissophyllum, Mentha spp, Menyanthes trifoliate, Mespilus germanica, Mirabilis jalapa, Moringa oleifera, Morus alba, Myosotis sylvatica, Myrrhis odorata, Myrtus communis, Narcissus tazetta, Nasturtium officinale, Nelumbo nucifera, Nepeta cataria, Nicotiana glauca, Nicotiana tabacum, Nigella sativa, Nuphar lutea, Nymphaea alba, Ocimum basilicum, Oenanthe aquatic, Oenothera biennis, Olea europaea, Ononis spinosa, Origanum majorana, Origanum vulgare, Ornithogalum umbellatum, Oxalis acetosella, Oxalis pes-caprae, Paeonia officinalis, Panax ginseng, Panicum miliaceum, Papaver rhoeas, Papaver somniferum, Parietaria officinalis, Parthenocissus quinquefolia, Passiflora incarnate, Pastinaca sativa, Paulinia sorbilis, Petroselinum spp, Peucedanum ostruthium, Peumus boldus, Phragmites australis, Physalis alkekengi, Phytolacca decandra, Picea excelsa, Pimpinella anisum, Pinus mugo, Pinus sylvestris, Piper nigrum, Plantago lanceolata, Plantago Major, Plantago psyllium, Polygala vulgaris, Polygonum aviculare, Polygonum bistorta, Polygonum hydropiper, Portulaca oleraceae, Potentilla reptans, Primula spp, Prunella vulgaris, Prunus spinosa, Pulmonaria officinalis, Punica granatum, Quercus ilex, Quercus palustris, Quercus robur, Quercus rubra, Ranunculus bulbosus, Ranunculus ficaria, Ranunculus sceleratus, Raphanus raphanistrum, Rhodiola rosea, Ribes nigrum, Ribes rubrum, Ricinus communis, Robinia pseudoacacia, Rosa canina, Rosmarinus officinalis, Rubus idaeus, Rubus ulmifolius, Rumex acetosa, Rumex acetosella, Rumex conglomerates, Rumex crispus, Rumex obtusifolius, Ruscus aculeatus, Ruta graveolens, Saccharomyces cerevisiae, Salix alba, Salix babylonica, Salix cinerea, Salvia pratensis, Salvia spp, Sambucus nigra, Sanicula europaea, Saponaria ocymoides, Saponaria officinalis, Satureja spp, Scilla bifolia, Scrophularia nodosa, Scutellaria galericulata, Sedum maximum, Sempervivum tectorum, Senecio vulgaris, Silybum marianum, Sinapis spp, Smilax aspera, Solanum dulcamara, Solanum melongena, Solanum nigrum, Sonchus asper, Sorbus aucuparia, Sorbus domestica, Stachys palustris, Stellaria aquatica, Stellaria media, Symphytum officinalis, Syringa vulgaris, Tanacetum vulgare, Taraxacum officinale, Taxus baccata, Tecoma impetiginosa, Teucrium chamaedrys, Theobroma cacao, Thuja occidentalis, Thymus spp, Tilia platyphyllos, Tilia spp, Tragopogon pratensis, Trifolium pretense, Trifolium repens, Trigonella foenum-graecum, Triticum aestivum, Tropaeolum majus, Tussilago farfara, Typha latifolia, Ulmus minor, Umbilicus rupestris, Uncaria tomentosa, Urtica dioica, Urtica spp, Vaccinium myrtillus, Valeriana officinalis, Valerianella locusta, Verbascum sinuatum, Verbascum Thapsus, Verbena officinalis, Veronica anagallisaquatica, Veronica persica, Vinca minor, Viola odorata, Viola tricolor, Vitex agnus-castus, Xanthium strumarium, Zea mays, Zingiber officinalis, Ziziphus jujube.*

The present disclosure also concerns methods of characterizing an activity or function of miRNA identified in a previous step, within a cell. In some embodiments, a method comprises:

a) introducing into one or more cells a synthetic or non-synthetic miRNA plant molecule that corresponds to a miRNA sequence and

b) comparing one or more characteristics of cell(s) having the RNA molecule with cells in which the synthetic miRNA molecule has not been introduced.

In certain embodiments, the cells with the synthetic miRNA may be compared to cells in which a different molecule was introduced (such as a negative control that does not include a miRNA region or such as a positive control that includes a miRNA with known anti-tumour properties - human miR-34a). It is contemplated that the compared cells need not be evaluated at the same time. In fact, the comparison cells should not have been necessarily cultured at the same time; one may refer to a report or previous observation.

In the preferred embodiment miRNA-mRNA cross-kingdom interactions are tested in Jurkat and THP1 cell culture models. The choice of cell models is based on authors' observation that Jurkat and THP1 cells have low levels of expression of *hsa-miR-34a* compared to HepG2, HT29 (human colorectal adenocarcinoma), Caco-2 (human colon epithelial cancer) e FPAF cell lines.

In a first step human Jurkat E6-1 lymphoid and THP1 monocytoid cell lines (American type Culture Collection, USA) are grown in suspension culture at a density of $7 \times 10^5$ cell/ml, and $0.4 \times 10^5$ cells/ml, respectively. Human peripheral blood mononuclear cells (PBMCs) are used as negative control. PBMCs from healthy donors are separated by density gradient according to the standard technique by Fycoll Hypaque (Lonza, USA) and cultured at a density of $10^6$ cell/ml. Jurkat and THP1 cell lines and primary PBMCs are cultured in Roswell Park Memorial Institute (RPMI) 1640 medium (Invitrogen, USA), supplemented with 10% fetal bovine serum (FBS, Invitrogen), 2 mM glutamine (Hyclone, UK), 50 U/mL penicillin and 50 U/mL streptomycin (Hyclone). All the cell lines are cultured at 37°C under humidified 5% $CO_2$ atmosphere.

In the second step the said cells are transfected with synthetic human miR-34a (*hsa-miR-34a*) purchased from Qiagen, USA and *plant-miR-20* purchased from Qiagen, USA, by lipofectamine method according to manufacturer's instructions (Hi-Fect, Qiagen, USA). FITC tagging of miRNAs is used for control of transfection. Cells are harvested 72h after transfection and characterized for the efficiency of transfection by flow cytometry analysis and for the effect of miRNAs on specific target gene by Western Blot.

In the third step cell viability assay is performed: Percentage or absolute number of dead/living cells is evaluated using the trypan blue dye exclusion test (Euroclone S.P.A. ITA).

In the fourth step apoptosis assay is performed: 72h post-transfection apoptosis is assessed through flow cytometry analysis of isolated nuclei stained with propidium iodide (PI), carried out on a Becton Dickinson FACS analyzer (BD Biosciences, USA).

In the fifth step intracellular Bcl-2 staining is performed: To determinate Bcl-2 intracellular expression 72h upon transfection cells were harvested, fixed and permeabilized with 70% ethanol and incubated with PE-conjugated anti-human Bcl-2 (BD Biosciences, USA). Stained cells are analyzed by flow cytometry analysis Becton Dickinson FACS analyzer (BD Biosciences, USA).

In an embodiment SIRT-1 expression and Bcl-2 expression are analyzed using Real time PCR (CFX96 Real-Time System instrument, Bio-Rad, CA, USA) according to SYBR Green protocol. An amount of 0.25 micrograms of DNase-treated RNA from each sample is reverse-transcribed in a total volume of 20 μl with high-capacity cDNA Reverse Transcription Kits (Applied Biosystem by Life Technologies NY, USA), according to the manufacturer's instructions. Real-time PCR reaction includes 0.20 μl of cDNA, 200 nM of each primer, 12.5 μl of SYBR ® Green PCR Master Mix (Applied Biosystem), in a total volume of 25 μl and is conducted for 1 cycle at 95°C for 5 minutes and then for 40 cycles of 95°C for 10 sec, 60°C for 15 sec. Each sample is analyzed in triplicate technical replication and a negative control (no template reaction) is included to check out any possible artefact. The housekeeping gene Beta-actin is used to normalize the results. Each reaction is completed with a melting curve analysis. When primer pairs show a single peak, the specificity of amplification and the lack of non-specific products and primer dimers is confirmed. Quantification is performed using the threshold cycle (Ct) comparative method according to the MIQE guidelines [58]. The following primers are used: SIRT-1 specific 5'-TTCGCTCTTTTCCTCCGTCC-3' and 3'-CAGCGTGTCTATGTTCTGGGT-5'; Bcl2 specific 5'TCCCTCGCTGCACAAATACTC-3' and 3'-ACGACCCGATGGCCATAGA-5'; and Beta-actin specific 5'-GCACTCTTCCAGCCTTCC-3' 3'-AGGTCTTTGCGGATGTCCAC-5' (BIOFAB, Rome Italy).

In an embodiment SIRT-1 expression and Bcl2/BAX expression are analyzed using Western blot analysis. Cells from the second step are suspended in buffers for cytoplasmic and nuclear proteins extraction and processed for Western blot analysis. The primary antibodies used are the rabbit monoclonal anti-SIRT1 and anti-Bcl2, mouse monoclonal antibody BAX and the goat monoclonal antibody human beta-actin (Santa Cruz biotechnology, CA USA). The secondary antibodies used are anti-goat, anti-mouse and anti-rabbit IgG chain specific conjugated to peroxidase (Calbiochem, Merck Millipore, Darmstadt, Germany).

The authors confirmed that tumour suppressor activity of selected plant miRNA is comparable to synthetic human miR-34a (*hsa-miR-34a*) activity in *in vitro* assays with leukemia cells, as described in detail in the experimental section of the present description.

Therefore, the nutraceutical use of plant miR-20 for treatment of leukemia is an object of the present invention.

In some embodiments the plant composition or extract produced by the methods described herein is incorporated into a food product to produce an enriched food product. The term "food product" as used herein refers to any substance containing nutrients that can be ingested by an organism to produce energy and maintain life. In some embodiments, the plant composition or extract produced by the methods described herein is used in the preparation of enriched food products comprising adequate amounts of plant miRNAs. The term "enriched food product" as used herein refers to a food product that has been modified to include the plant composition or extract produced by the methods described herein, which provides a benefit such as a leukemia preventing/treating property beyond the basic function of supplying nutrients. Furthermore, the term "food product" as used herein refers to nutrients recommended in the diet for leukemia patients prior to or both during and after chemotherapy or radiotherapy. The composition may be used as a supplement to an individual's diet, however, it may also be designed to provide complete nutritional support.

The plant composition or extract produced by the methods described herein can be incorporated into any food product. Exemplary food products include, but are not limited to, baked goods (cakes, cookies, crackers, breads, scones, muffins and biscuits), liquid foods (e.g. soups and liquid supplements such as multifibre drinks) and dairy-type products (including, but not limited to yogurt, custards, rice pudding, mousses, ice cream), desserts (including, but not limited to, sherbet, sorbet, water- ices, granites and frozen fruit purees), pasta products, rice products and other cereal products, meal replacement products, nutrition bars, trail mix, granola, beverages (including, but not limited to smoothies, water or dairy beverages, and soy- or rice-based beverages), breakfast-type cereal products such as oatmeal. For beverages, the plant composition or extract (or miRNAs isolated from the plant composition or extract) may be in solution, suspended, emulsified or present as a solid.

In one embodiment, the enriched food product is a meal replacement product. The term "meal replacement product" as used herein refers to an enriched food product that is intended to be eaten in place of a normal meal. Nutrition bars and beverages that are intended to constitute a meal replacement are types of meal replacement products.

In another embodiment, the food product is a dietary supplement. The term "dietary supplement" as used herein refers to a substance taken by mouth that contains a "dietary ingredient" intended to supplement the diet. The term "dietary ingredient" includes, but is not limited to, the mRNAs as disclosed herein, as well as vitamins, minerals, herbs or other botanicals, amino acids, and substances such as enzymes, organ tissues and metabolites. The dietary supplement may be solid formulations, such as capsules or tablets, or liquid formulations, such as solutions or suspensions. The nutraceutical compositions according to the present invention may further contain protective hydrocolloids (such as gums, proteins, modified starches), binders, film-forming agents, encapsulating agents/materials, wall/shell materials, matrix compounds, coatings, emulsifiers, surface active agents, solubilising agents (oils, fats, waxes, lecithins etc.), adsorbents, carriers, fillers, co-compounds, dispersing agents, wetting agents, processing aids (solvents), flowing agents, taste-masking agents, weighting agents, jellifying agents, gel-forming agents, antioxidants and antimicrobials.

In yet another embodiment, the food product is a medical food. The term "medical food" as used herein means a food which is formulated to be consumed or administered entirely under the supervision of a physician and which is intended for the specific dietary management of a disease or condition for which distinctive nutritional requirements, based on recognized scientific principles, are established by medical evaluation. The nutraceutical compositions according to the present invention may be in any galenic form that is suitable for administering to the body, especially in any form that is conventional for oral administration, e.g. in solid forms such as tablets, pills, granules, dragees, capsules and effervescent formulations, such as powders and tablets, or in liquid forms, such as solutions, emulsions or suspensions as e.g. beverages, pastes and oily suspensions. The pastes may be incorporated in hard or soft-shell capsules, whereby the capsules feature e.g. a matrix of (fish, swine, poultry, cow) gelatin, plant proteins or lignin sulfonate. Examples for other application forms are those for transdermal, parenteral or injectable administration. The dietary and pharmaceutical compositions may be in the form of controlled (delayed) release formulations.

If the nutraceutical composition is a pharmaceutical formulation the composition further contains pharmaceutically acceptable excipients, diluents or adjuvants. Standard techniques may be used for their formulation, as e.g. disclosed in Remington's Pharmaceutical Sciences, 20th edition Williams & Wilkins, PA, USA. For oral administration, tablets and capsules are preferably used which contain a suitable binding agent, e.g. gelatin or polyvinyl pyrrolidone, a suitable filler, e.g. lactose or starch, a suitable lubricant, e.g. magnesium stearate, and optionally further additives. Preferred are formulations containing the concentration from the range of 0.1mg to 1000mg of the active miRNAs per administration unit, e.g. per tablet or capsule.

It is contemplated that a therapeutic benefit includes, but is not limited to, a decrease in pain, a decrease in morbidity, a decrease in a symptom. For the present invention, with respect to cancer, it is contemplated that a therapeutic benefit from administration of said plant miRNA homologue molecules, can be inhibition of tumor growth, prevention of metastasis, reduction in number of metastases, inhibition of cancer cell proliferation, induction of cell death in cancer cells, inhibition of angiogenesis near cancer cells, induction of apoptosis of cancer cells, reduction of cancer cell viability or number of viable cancer cells, reduction in pain, reduction in risk of recurrence, induction of chemo- or radiosensitivity in cancer cells, prolongation of life, and/or delay of death directly or indirectly related to cancer.

Aside from applications for humans, the compositions of this disclosure have additional uses in the veterinary world. Animals that can benefit from active miRNA tumour suppressing properties include those animals which are subject to following types of cancer: bladder cancer, bone cancer, liver cancer, carcinoma, brain tumor, lung cancer, mouth cancer, skin cancer, lymphoma, leukemia, lymphosarcoma, osteosarcoma, and stomach cancer. Preferred "animals" are pets or companion animals and farm animals. Examples of pets are dogs, cats, birds, aquarium fish, guinea pigs, (jack) rabbits, hares and ferrets. Examples of farm animals are aquaculture fish, pigs, horses, ruminants (cattle, sheep and goats) and poultry.

Examples aiming at better illustrating the methods disclosed in the present description arc reported below; such examples are in no way to be considered as a limitation of the preceding description and of the subsequent claims.

EXAMPLES

Example 1: Identification of specific plant miRNAs sharing important functional sequence homologies with human miRNAs targeting specific cancer related proteins

Step 1

In the first step, the miRNAs with a putative active role within cross-kingdom post-transcriptional regulation are selected using an algorithm able to compare libraries of microRNAs belonging to organisms from plant and animal kingdoms. To validate MirCompare algorithm and select a set of plant miRNAs with a predictable active role in cross-kingdom, *O. europaea* and *H. sapiens* mirnomes are compared.

In the analysis of 172 *O. europaea* [40] and 2042 *H. sapiens* microRNAs (www.mirbase.org) [59], a total of 351,224 different comparisons is generated (Figure 1A). Exclusion of those with an r value lower than 0.45, allows for the reduction of the number to 12,134. After the second filtering phase over the seed region, 2,164 different comparisons are obtained involving 117 *O. europaea* and 1,001 *H. sapiens* different microRNAs.

Step 2

In the second step, identification of *O. europaea* miRNAs targeting human mRNAs is performed. In order to experimentally validate MirCompare algorithm, a set of genes that could be both regulated by exogenous miRNAs through the host endogenous machinery is identified. The COMIR algorithm [48] is used to extract a list of mammalian genes potentially regulated by *O. europaea* miRNAs. Next, Diana Tarbase [57] is used to select only the experimentally validated miRNA-mRNA interactions.

The *plant-miR-20* identified in this step, shows a putative functional homology with *hsa-miR-34a* (Figure 1B). A variety of solid tumours and haematological malignancies (in particular leukemia) are correlated with silenced expression of *hsa miR-34a* [60, 61].

Step 3

In order to evaluate the interaction strength between *plant-miR-20* and *hsa-miR-34a* and their targets that have been cross-validated using the COMIR-TarBase double-step method, the free energy variation in miRNA-mRNA duplex formation is measured. Since miRanda software finds multiple pairing regions for each miRNA-mRNA couple, only the most negative free energy value is reported. As the number of pairing regions varies from pair to pair, no Standard Deviation value is assigned. *plant-miR-20* shows high propensity to bind NOTCH 1, SIRT1, Bcl-2, MYC and RRAS genes as well as their human homologous *hsa-miR-34a* (Table 1).

Table 1.

| | ΔG (kcal/mol) | |
|---|---|---|
| | hsa-miR-34a | plant-miR-20 |
| SIRT1 | -32.88 | -22.31 |
| RRAS | -21.67 | -15.59 |
| BCL2 | -19.6 | -13.5 |
| NOTCH 1 | -22.79 | -8.61 |
| MYC | -5.42 | -11.4 |

Example 2: Effectiveness of plant miRNA mimics as anti-leukemia agents

Step 1

To evaluate whether *plant-miR-20* mimics inhibit the SIRT-1 and BCL-2 protein expression as well as, or more efficiently than *hsa-miR-34a* does, transfection with synthetic (mimic) plant-miR-20 and *hsa-miR-34a* (purchased from Qiagen, USA) into lymphoid (Jurkat), monocytoid (THP-1) cell lines and in PBMCs from healthy donors is performed.
The efficiency of transfection is assessed by monitoring percentage of fluorescent positive (FL1- positive) cells by flow cytometry analysis seventy-two hours post transfection (Figure 2A). Cell lines and PBMCs treated with non-transfected Hi-Fect control vehicle (HF) show a Mean Intensity Fluorescence (MIF) lower than $10^2$ and cells are considered FL1-positive when MIF is higher than $10^3$. Following this criterion, the percentage of FITC-positive Jurkat and THP1 cells as well as PBMCs is significantly higher than HF control cells demonstrating the possibility to transfect synthetic plant miRNAs into human cell lines. The results of Figure 2B represent the mean values ± SD of the percentage of FL1-positive cells obtained from three independent biological experiments.

Step 2

In the next step mRNA and protein expression of SIRT1 and Bcl-2, specific targets of *hsa-miR-34a* regulation are investigated in order to confirm the effect of miRNAs treatment. The transfection of *plant-miR-20* mimic does not affect the levels of SIRT-1 and Bcl-2 mRNA (Figure 3A and D) but determines a significant decrease of SIRT-1 and Bcl-2 proteins compared with control (Figure 3B and E, $p < 0.05$ for all treatment vs CTR-HF cells) in Jurkat and THP-1 cell lines. The transfection with *plant-miR-20* mimic is found to be more effective in decreasing the expression of human SIRT 1 protein expression compared to *hsa-miR-34a* ($p < 0.01$ *plant-miRNAs* vs *hsa-miR34a*). Moreover, the transfection in PBMCs has no effects on the modulation of these two proteins (Figure 3C and F).

Step 3

In the third step *plant-miRNAs* mimics transfection on cell viability and apoptosis is evaluated. Mimics miRNAs transfection determines a significant decrease of cell number after 72 hours (Figure 4A, $p<0.05$ for all treatment vs CTR-HF cells) in Jurkat and THP-1 cell lines and a significant inverse correlation between the percentage of FITC-positive cells analysed by flow cytometry after transfection and the decrease of cell number count assessed by trypan blue assay (Figure 4B) suggesting a direct effect of *plant-miRNA* on host cell viability. No effect on cell viability is reported on PBMCs transfected.
The treatment of Jurkat cells with the different mimics shows a significant increase of apoptosis in comparison with control samples (Figure 5A). THP-1 analysis confirms the results observed in Jurkat cells while in PBMCs from healthy donors no significant difference in the apoptosis levels is observed.
In order to confirm that the apoptosis is directly induced by miRNAs the expression of the intracellular anti-apoptotic protein BCL-2 (a specific target of *hsa-miR-34a* regulation) is investigated. The treatment of Jurkat and THP-1 cells with mimics derived from *plant-miRNA* sequences (Figure 5B) shows a significant decrease of Bcl-2-positive cells in comparison with control samples.

Alternatively, apoptosis is characterized by western blot analysis for the expression of BAX protein involved in intrinsic signalling pathways of programmed cell death (Figure 5C). Cells are solubilized at 4°C in lysis buffer (50mM Tris-HCL pH 7.4, 1mM EDTA, 1mM EGTA pH 7.4, 1% Triton-X, 150mM NaCl, 0.25% sodium deoxycholate, 1% NP-40 and, freshly added, 1mM PMSF, 5 mM DTT, 1 mg/ml leupeptin, 1 mg/ml pepstatin, 2 mg/ml aprotinin, 1mM Na3VO4, 20mM Na3F, all purchased from Sigma, Italy) and loaded onto a 10% SDS-polyacrylamide gel, subjected to electrophoresis and transferred to nitrocellulose membrane (Bio-Rad laboratories, Hercules, CA, USA). After blocking the membrane in 20% non-fat dried milk and 3% BSA in TTBS (20mM Tris-HCl pH 8.0, 0.9% NaCl, 0.03% Tween 20, all purchased from Sigma, Italy), the blots are incubated overnight at 4°C with diluted primary antibody and, subsequently, washed and then incubated with anti-mouse or anti-rabbit IgG chain-specific conjugated to peroxidase (Calbiochem, Merck Biosciences, Darmstadt, Germany). Binding of antibodies is detected by chemiluminescence staining using the ECL detection kit (Amersham Biosciences, Little Chalfont, UK). The following antibodies are used: rabbit polyclonal antibodies against human SIRT 1 (1:1000) and mouse anti human b-actin (1:5000) Santa Cruz Biotechnology, Santa Cruz, CA USA). Comparative analysis of the bands is performed by quantitative densitometry using the Tina software (version 2.10, Raytest, Straubenhardt, Germany).

Example 3: Identification and quantification of *plant-miR-20* miRNA in *Moringa* plant tissues

To verify the presence and determine the expression levels of *plant-miR-20* like miRNAs in *M. oleifera* leaves (F), immature seed (SV) and mature seed (SS) tissues, qRT-PCR is performed. The seeds from *M. oleifera* trees are collected at pods maturity before they split open and fall to the ground. Germination is performed in a greenhouse by placing the seeds on paper soaked in sterile water. Total RNA is isolated from defined plant tissue by using mirVana kit (Ambion, USA), according to the manufacturer's protocol. RNA quality and quantity are evaluated by Agilent 2100 Bioanalyzer (Agilent Technologies, Waldbroon, Germany) and by Spectrophotometer (SmartSpec Plus, Bio-Rad, USA). The expression level of different miRNAs is validated using poly(A)-tailed qRT-PCR method. According to the manufacturer's protocols (Exiqon A/S, Vedbaek, Denmark), a poly-A tail is added to the mature miRNA templates (20ng). cDNA is synthesized using a poly-T primer with a 3' degenerate anchor and a 5' universal tag at 42°C for 60 min followed by heat-inactivated for 5 min at 95°C. To provide a control for the quality of the cDNA synthesis reaction and the PCR, RNA spike-in (UniSp6) is added to the sample prior to cDNA synthesis. The cDNA template is then amplified using miRNA-specific and LNA™-enhanced forward and reverse primers. SYBR®Greenis used for detection. The reactions are carried out in an Rotor-Gene®Q 72-Well Rotor (Qiagen, USA) with the following amplification conditions: activation/denaturation at 95°C for 10 min followed by 40 cycles of denaturation at 95°C for 10s, annealing and extension together at 60°C for 60 sec. Finally, melting analysis is performed to confirm the absence of false-positive peaks. Two controls (no template control and no Reverse Transcription control) are included in all reactions. qRT-PCR for *plant-miR-20* confirmed the presence of this miRNA in *M. oleifera* plant tissues (mature and immature seeds and leaves) (Figure 7A). Each value represents the mean of three different biological replications. Further, absolute concentrations of *plant-miR-20* in *M. oleifera* tissues (mature seeds-SS, immature seeds-SV, leaves-F) are determined as number of copies of each miRNA per one microliter of plant tissue extract. Plant tissues are further subject to different conditions: boiled for 15 minutes (15), boiled for 2 hours (2h) and kept at -80°C for 24 hours (24h) (Figure 7B).

Example 4: Effect of Moringa plant extracts containing plant-miRNA on leukemia cell proliferation

Step 1

In the first step *M. oleifera* plant extracts containing *plant-miR-20* are prepared as follows. Plant tissues (leaves, immature and mature seeds) are collected as described in Example 3 and two sets of extracts are prepared. First set of extracts is maintained at -80°C prior to use for cell treatment. Second set of extracts is boiled at 100°C prior to use for cell treatment. Following concentrations for all soluble extracts are prepared: 0, 0.01, 0.1, 0.25, 0.5, 1, 2.5, 5 and 10 mg/ml.

Step 2

In the second step THP1, Jurkat cell lines and PBMCs are treated for 72 hours with the extracts from the first step and evaluation of cell proliferation (IC50, 50% of inhibition) is performed. The IC50 is calculated for each set of extracts (boiling and freezing preparations) of *M. oleifera* leaves, immature and mature seeds. Authors demonstrated a greater sensitivity of Jurkat cells to the treatment with all preparations compared to THP-1 and PBMCs (Table 2). For the leaves preparations, a fresh weight concentration (FWC) between 1.8-2 mg/ ml was sufficient to inhibit 50% of cell proliferation in Jurkat cells, whereas for the THP1 that the PBMCs concentrations between 5.8-7 mg/ml of FWC are necessary to achieve the same results. This is confirmed also for treatments of Jurkat cells with both immature and mature seeds where, for all preparations, the concentration range for inhibiting 50% of cell proliferation was between 1.5 to 2.6 mg/ml for immature and between 1.6 and 2.7 for mature seeds. THP1 and PBMCs appear less sensitive to the treatments, in fact, it was necessary to use a concentration greater than 2-3 times. The preparation -80°C for all parts of the plant proved to be significantly more toxic in both cell lines analyzed: the IC50 values for leaves as well as immature and mature seeds were considerably lower than the preparations made by boiling (Table 2).

Table 2: IC50 values on cell proliferation after 72 hours of *M. Oleifera* treatment

**a) LEAVES**

| mg/ml | 100°C | -80 °C |
|---|---|---|
| THP1 | 6,86±0,05 | 5,38±0,038* |
| JURKAT | 2,55±0,04++ | 1,41±0,04**** |
| PBMCs | 6,31±0,15 | 6,69±0,25 |

**b) IMMATURE SEEDS**

| | 100°C | -80 °C |
|---|---|---|
| THP1 | 8,10±0,05 | 2,80±0,03** |
| JURKAT | 2,62±0,04++ | 1,51±0,03**** |
| PBMCs | 7,31±0,18 | 4,65±0,14** |

**c) MATURE SEEDS**

| | 100°C | -80 °C |
|---|---|---|
| THP1 | 5,37±0,04 | 3,24±0,01** |
| JURKAT | 2,71±0,27++ | 2,03±0,16++ |
| PBMCs | 10,00±0,04 | 2,32±0,27** |

*p<0,05 **p<0,001 -80°C preparation vs other preparations
+ p<0,05 ++ p<0,001 Jurkat cells vs THP1 or PBMCs

Taken together, these data demonstrate that Jurkat cells are the most sensitive to treatment with aqueous extracts of *M. oleifera* in terms of proliferation, and the preparation of mature seeds obtained by freezing at -80°C appeared more toxic than the other preparations.

Step 3

In the third step THP1, Jurkat cell lines and PBMCs are treated for 72 hours with the extracts from the first step and evaluation of apoptosis by analyzing the percentage of hypodiploid nuclei using propidium iodide (PI) staining and flow cytometry analysis is performed. The evaluation of apoptosis after 72 hours of treatment with the first set of extracts (freezing) underlined toxic effects on both cell lines analyzed and on the lymphocytes from healthy donors (Figure 8A, B and C) therefore first set of extracts was not used for further investigations.

Treatment with leaf extracts from second set (boiling) (Figure 9A) induced a 20% increase of hypodiploid nuclei in THP1 starting from a fresh weight concentration of 0,5 mg/ml, while in Jurkat a concentration of 1mg/ml of *M. oleifera* produced the same effect. The PBMCs appeared to be less sensitive: 2.5 mg/ml of fresh weight *M. oleifera* concentration was required to have a 25% of apoptosis.

Treatment with immature seed extracts from second set (Figure 9B) induced significant apoptosis levels at fresh weight concentrations of 2.5mg/ml in THP1. The Jurkat cells were not found to be sensitive to these treatments while PBMCs from healthy donors were susceptible as well as THP1.

Treatment with mature seed extracts from second set (Figure 9C) induced a significant increase of apoptosis at low concentrations (0.25 mg/ml) in THP-1 cells. In the Jurkat cells a significant apoptosis increase was observed at concentrations of 1mg/ml while in PBMCs never exceeded 10% even at the higher concentration (Figure 9C).

Taken together, these data demonstrate that mature seed extracts from the second set show selective pro-apoptotic effect on tumour cells compared to PBMCs of healthy donors. In fact in THP1 and Jurkat cell lines 2.5 mg/ml determined a significant increase of apoptosis while in PBMCs never exceeded 10% even at the higher concentration.

Step 4

In the fourth step the increased apoptosis level in cells induced by treatment with 1mg/ml of mature seeds extract from second set, is further characterized by western blot analysis of SIRT-1 and Bcl-2 protein expression. Results show a significant decrease of SIRT-1 and Bcl-2 proteins in Jurkat and THP-1 cell lines, while in PBMCs from two healthy donor the *M. oleifera* treatment does not induce any SIRT-1 and Bcl2 protein expression modification (Figure 10A and B). A significant increase of apoptosis levels in tumor cell lines as a consequence of Bcl-2 and SIRT-1 down regulation protein expression is demonstrated (Figure 10A and B). PBMCs of healthy donors resulted particularly resistant to this treatment - at the FWC of 1mg/ml Jurkat and THP1 cell lines show an increase of apoptotic levels, while in PBMCs this effect is not observed (Figure 10B).

Example 5: Preparation of different *Moringa* extracts and aliments (pasta and granular soup) containing specific miRNA.

*M. oleifera* extracts containing *plant-miR-20* described in Example 4 are prepared by grounding the seeds with pestle, mortar and liquid nitrogen and then boiling the powder material in bidistilled water (1mg/ml) at 100°C for 1 hour. Extracts are subsequently filtered (using 0.22 $\mu$m diameter mash) and stored at -20°C prior to use.

The main raw material used for the production of pasta is flour of durum wheat. Minor ingredients - *M. oleifera* aqueous extracts and the aromas are dehydrated and delivered in concentration of 7 to 25%. Flours are subjected to a filtration step using filter and magnets that eliminate impurities and metallic substances, before the production process for obtaining pasta and granular soup. The semolina, free of foreign bodies, is conveyed via a suction-compression system equipped with rotary valves to the measuring device, which allows adjusting the flow rate of the semolina to be sent in the dough. The water is added to the mixture through pipelines and the dispenser (usually 20-25% for processing on the press). The addition of flavorings (in percentage of 2%) is performed either by automatic or manual mode. The semolina and water, suitably dosed, are amalgamated by means of mechanical shovels. The dough, by means of rotary valves, passes into the section of the vacuum, where incorporated air is sucked out (approximately 70% for the sizes bronze, about 90% for the sizes Teflon), in order to make the mixture more homogeneous. The mixture obtained is pushed, by means of screws without end, to the press machine which gives shape to the dough. The length of the format is defined by the horizontal cut made by the knives in stainless steel placed below the drawing plates. After the extrusion the short pasta goes to the pre-wrapping (shaker) traveling on frames made of materials suitable for contact with food, while the long pasta is positioned on reeds in steel in specially protected shelves. The paste passes through a pre-wrapping for a first drying. The temperature of the chamber is of 55-60 degrees with a relative humidity of 18-20 degrees and a residence time of 4-5 minutes. The dough undergoes a first drying and is poured on frames. For filling of the drying chamber (when possible) for short pasta 8 trolleys are formed - with 24 frames each, while for the long pasta canes are positioned on 2 carts on 6 floors.

Drying takes place at 12 cells at a temperature and humidity controlled according to drying diagrams preselected recipes:

- Long delicate (eg. Scialatielli, lasagna)

- Long delicate (eg. Spaghetti, bucatini)

- Festonate (eg. Ricciarelle, pappardelle)

- Short

In particular, recipes involve following pasta shapes: Calamarata, Lumaconi, Paccheri Ondulati, Radiatori, Penne Zite Lisce, Conchiglioni, Mezzi Paccheri Lisci, Paccheri Rigati, Scialatielli, Penne Lisce, Eliconi, O'Sole Mio, Pennoni Lisci, Strozzapreti, Pennette Rigate, Lasagnacce, Paccheri Lisci, Pennoni Rigati, Conchigliette, Rigatoni, Tubetti Lisci, Creste di gallo, Torchietti, Tubettoni Rigati, Penne Prisma, Maccheroni, Lumache Medie, Tubetti Rigati, Tubettoni Lisci, Penne Rigate, Gemelli, Penne Zite Rigate, Gramigna, Amorini, Candele, Caserecci, Ruote, Fusilli, Pasta Mista, Cannelloni, Fusilli Napoletani Lunghi, Ricciarelle, Mezzanini, Bucatini, Mezze Candele, Fusilli Napoletani Corti, Spaghetti N.7, Lasagna, Tagliatelle, Capunti, Ferretto Calabro, Linguine, Spaghetti alla Chitarra, Tagliatelle a Nido, Cavatelli, Pappardelle, Penne Candela, Spaghetti N.5, Pappardelle a Nido, Fusilli Pugliesi, Fusilli Avellinesi, Orecchiette, Lumaconi 3 Colori, Spaghetti Peperoncino, Fusilli Cilentani, Trofie, Pennoni 3 Colori, Spaghetti 3 Colori, Filei Tropeani, Conchiglioni 3 Colori, Pennette 3 Colori, Spaghetti Nero Seppia, Caserecci, Ruote, Fusilli, Pasta Mista, Cannelloni, Foglie di Carciofo, Gigli 3 Colori, Cuori 3 Colori.

Depending on the type of diagram values within the following ranges may be assumed:

- Temperature from 40 to 66 ° C

- Humidity from 50 to 90 ° R.H.

- Time from 450 to 1300 minutes.

When the cell is filled completely, a new phase of condition preparation is carried out.

The Program Logic Control (PLC) allows monitoring in real time the progress of the drying which consists of adding moisture in a first stage and then delete it in a second phase to reach the pre-set value (12.5°). At the end of the drying process, the cells are open and the product is left for a few minutes before unloading the cells.

Once the drying phase is finished, the short pasta is poured from the frames in plastic bins suitable for food use, whereas long pasta is removed manually from the trolleys and poured in boxes with polyethylene bags suitable for contact with food. Later, pasta is covered with sheeting fabric suitable for contact with food, stored in the warehouse and stacked according to size. Cartons of long pasta are placed on pallets, weighed, identified with the same sign of short pasta and taken to the packaging department in a dedicated area adjacent to the packaging lines long pasta.

In the final step the debittered moringa seed flour (DBMSF) (10%, 20% and 30%) and the extruded pasta products are evaluated for quality characteristics such as cooking quality, colour, texture, farinograph and micro-viscoamylograph. The proximate analysis indicates that the *Moringa* pasta has the highest overall quality compared to substituted blended samples but the protein content and dietary fiber is high in blended samples. The pasta shows cooking loss (4.326) when compared to debittered *Moringa* seed flour (10%, 20% and 30%) in the pasta formulations about 5.01, 4.28 and 3.97 respectively. The increased concentration of DBMSF reduces the cooking quality. Better results are obtained in semolina pasta and 10% DBMSF blend. The analysis of the pasta samples for the color also indicates that the 30% blended DBMSF has a slightly more color (61.60) than the durum semolina samples. The durum pasta has good texture and good cooking quality. By using debittered *Moringa* seed flour sample, it is possible to enhance the nutritional quality of pasta, without affecting its cooking and textural properties.

Example 6: Preparation of formulations with *Moringa* extracts containing *plant miR-20.*

In a preferred formulation *M. oleifera* extracts containing *plant-miR-20* described in Example 4 are prepared as described in Example 5. A preferred composition further contains pharmaceutically acceptable excipients, diluents or adjuvants. For anti-inflammatory effect the preferred composition will further include:

10-100mg acetylsalicylic acid

or 20-200mg ibuprofen

or 50-500mg paracetamol

and 40-400mg of extract of pineapple and pineapple stem

and 40-400mg dry extract of leaves and flowers *Spiraea*

and 40-400mg extract of leaves, flowers and roots of mallow

and 50-500mg dry extract of *Aloe vera*

and 500-1000mg dry extract of *Moringa* leaves.

and 500-1000mg of semi-dry extract of *Moringa.*

For anti-oxidant effect the preferred composition will further include:

100-1000mg vitamin C

or 50-500mg n -acetylcysteine

or 1-5 mg melatonin

or 1-10mg vitamin E

and 10-100mg of extract of grape fruit

and 1-10mg of polygonum root extract - resveratrol

and 10-100mg of extract of kiwi fruit

and 40-400mg extract of dry green tea leaves

and 50-500mg of extract of pomegranate fruit

and 50-500mg extract of cranberry fruit

and 50-500mg extract of fruit graviola

and 50-500mg extract of avocado fruit

and 30-300mg extract of carrot fruit

and 50-500mg extract of goji berries

and 500-1000mg of semi-dry extract of *Moringa*

and 500-1000mg dry extract of *Moringa* leaves.

For anti-cancer effect the preferred composition will further include:

50-500mg dry onion extract

and 50-500mg dry garlic extract

and 40-400mg extract of turmeric roots

and 40-400mg extract of dry green tea leaves

and 50-500mg extract of cranberry fruit

and 50-500mg extract of raspberries fruit

and 50-500mg / 1-10mg / 1-10mg extract of the fruit of tomatoes / lycopene / vitamin E, respectively

and 50-500mg extract of dry graviola fruit

and 500-1000mg of semi-dry extract of *Moringa*

and 500-1000mg dry extract of *Moringa* leaves.

The nutraceutical composition is to be taken on an empty stomach, meaning at a period of time at least two hours prior to consumption of any food or drink. Of course, an ordinarily skilled in the art will recognize that the amount of composition and frequency of use may vary from individual to individual. It is contemplated that a person may ingest less than one-half ounce, or more than ten ounces of the nutraceutical composition claimed in the present invention. Thus, the present invention contemplates the administration of one ounce, two ounces, three ounces or any volume of the formulations necessary to achieve the desired result including more than ten ounces per administration.

**Non-patent literature cited in the description**

1. Bartel DP. (2009) MicroRNAs: target recognition and regulatory functions. Cell.136 (2):215-33. doi: 10.1016/j.cell.2009.01.002

2. Huang Y, Shen XJ, Zou Q, Zhao QL. (2010) Biological functions of microRNAs. BioorgKhim. 36(6):747-52.

3. Liu Q, Wang F, Axtell MJ. (2014) Analysis of complementarity requirements for plant microRNA targeting using a Nicotianabenthamiana quantitative transient assay. The Plant cell. 26(2):741-53. doi: 10.1105/tpc.113.120972.

4. Mallory AC, Reinhart BJ, Jones-Rhoades MW, Tang G, Zamore PD, Barton MK, Bartel DP. (2004) MicroRNA control of PHABULOSA in leaf development: importance of pairing to the microRNA 5' region. The EMBO journal. 23(16):3356-64. doi: 10.1038/sj.emboj.7600340.

5. Xie M, Zhang S, Yu B. (2015) microRNA biogenesis, degradation and activity in plants. Cellular and molecular life sciences : CMLS. 72(1):87-99. doi: 10.1007/s00018-014-1728-7.

6. Brennecke J, Stark A, Russell RB, Cohen SM. (2005) Principles of microRNA-target recognition. PLoS Biol. 3(3).

7. Calin GA, Croce CM. (2006) MicroRNA signatures in human cancers. Nat Rev Cancer. 6(11):857-66.

8. Esquela-Kerscher A, Slack FJ. (2006) Oncomirs - microRNAs with a role in cancer. Nat Rev Cancer. 6(4):259-69.

9. Wiemer EA. (2007) The role of microRNAs in cancer: no small matter. Eur J Cancer. 43(10): 1529-44.

10. Esteller M (2011) Non-coding RNAs in human disease. Nature reviews Genetics 12(12):861 -874.

11. De Guire V, Robitaille R, Tetreault N, Guerin R, Menard C, Bambace N, Sapieha P. (2013) Circulating miRNAs as sensitive and specific biomarkers for the diagnosis and monitoring of human diseases: promises and challenges. Clinical biochemistry 46(10-11):846-860.

12. Broderick JA, Zamore PD. (2011) MicroRNA therapeutics. Gene therapy 18(12):1 104- 1 1 10

13. Mack GS. (2007) MicroRNA gets down to business. Nature biotechnology 25(6):631 - 638.

14. Lodygin D, Tarasov V, Epanchintsev A, Berking C, Knyazeva T, Körner H, Knyazev P, Diebold J, Hermeking H. (2008) Inactivation of miR-34a by aberrant CpG methylation in multiple types of cancer.Cell Cycle. 7(16):2591-600.

15. Gallardo E, Navarro A, Viñolas N, Marrades RM, Diaz T, Gel B, Quera A, Bandres E, Garcia-Foncillas J, Ramirez J, Monzo M. (2009) miR-34a as a prognostic marker of relapse in surgically resected non-small-cell lung cancer.Carcinogenesis.. 30(11):1903-9.

16. Chim CS, Wong KY, Qi Y, Loong F, Lam WL, Wong LG, Jin DY, Costello JF, Liang R. (2010) Epigenetic inactivation of the miR-34a in hematological malignancies. Carcinogenesis. 31(4):745-50.

17. Hermeking H. (2010) The miR-34 family in cancer and apoptosis. Cell Death Differ.17(2):193-9.

18. Ji Q, Hao X, Zhang M, Tang W, Yang M, Li L, Xiang D, Desano JT, Bommer GT, Fan D, Fearon ER, Lawrence TS, Xu L. (2009) MicroRNA miR-34 inhibits human pancreatic cancer tumor-initiating cells.PLoS One.4(8):e6816.

19. Liu C, Kelnar K, Liu B, Chen X, Calhoun-Davis T, Li H, Patrawala L, Yan H, Jeter C, Honorio S, Wiggins JF, Bader AG, Fagin R, Brown D, Tang DG. (2011) The microRNA miR-34a inhibits prostate cancer stem cells and metastasis by directly repressing CD44. Nat Med.17(2):211-5.

20. Bader AG. (2012) miR-34 - a microRNA replacement therapy is headed to the clinic. Front Genet. 3:120

21. Trang P, Wiggins JF, Daige CL, Cho C, Omotola M, Brown D, Weidhaas JB, Bader AG, Slack FJ. (2011) Systemic delivery of tumor suppressor microRNA mimics using a neutral lipid emulsion inhibits lung tumors in mice.MolTher. 19(6):1116-22.

22. Wiggins JF, Ruffino L, Kelnar K, Omotola M, Patrawala L, Brown D, Bader AG. (2010) Development of a lung cancer therapeutic based on the tumor suppressor microRNA-34.Cancer Res. 70(14):5923-30.

23. Craig VJ, Tzankov A, Flori M, Schmid CA, Bader AG, Müller A. (2012) Systemic microRNA-34a delivery induces apoptosis and abrogates growth of diffuse large B-cell lymphoma in vivo. Leukemia. 26(11):2421-4.

24. Chang TC, Wentzel EA, Kent OA, Ramachandran K, Mullendore M, Lee KH, Feldmann G, Yamakuchi M, Ferlito M, Lowenstein CJ, Arking DE, Beer MA, Maitra A, Mendell JT. (2007) Transactivation of miR34a by p53 broadly influences gene expression and promotes apoptosis. Mol Cell 26: 745-752.

25. Raver-Shapira N, Marciano E, Meiri E, Spector Y, Rosenfeld N, Moskovits N, Bentwich Z, Oren M. (2007) Transcriptional activation of miR34a contributes to p53-mediated apoptosis. Mol Cell 26: 731-743.

26. Corney DC, Flesken-Nikitin A, Godwin AK, Wang W, Nikitin AY. (2007) MicroRNA-34b and MicroRNA-34c are targets of p53 and cooperate in control of cell proliferation and adhesion-independent growth Cancer Res. 67(18):8433-8.

27. Hermeking H (2010) The mir 34 family in cancer and apoptosis. Cell Death Differ 17: 193-199

28. Li L, Yuan L, Luo J, Gao J, Guo J, Xie X (2013) Mir 34a inhibits proliferation and migration of breast cancer through down regulation of bcl 2 and sirt1. Clin Exp Med 13: 109-117

29. Lin Q, Mao Y, Song Y, Huang D (2015) MicroRNA-34a induces apoptosis in PC12 cells by reducing B-cell lymphoma 2 and sirtuin-1 expression. Mol Med Rep 12(4):5709-14. doi: 10.3892/mmr.2015.4185.

30. Matsushita N, Takami Y, Kimura M, Tachiiri S, Ishiai M, Nakayama T, Takata M (2005) Role of nad dependent deacetylases sirt1 and sirt2 in radiation and cisplatin induced cell death in vertebrate cells. Genes Cells 10: 321-332.

31. Castro RE, Ferreira DM, Afonso MB, Borralho PM, Machado MV, Cortez Pinto H, Rodrigues CM (2013) Mir 34a/sirt1/p53 is suppressed by ursodeoxycholic acid in the rat liver and activated by disease severity in human non alcoholic fatty liver disease. J Hepatol 58: 119-125.

32. Varkonyi-Gasic E, Gould N, Sandanayaka M, Sutherland P, MacDiarmid RM. (2010) Characterisation of micro-RNAs from apple (Malus domestica 'Royal Gala') vascular tissue and phloem sap. BMC Plant Biol. 10:159. doi: 10.1186/1471-2229-10-159.

33. Pulido A, Laufs P. (2010) Co-ordination of developmental processes by small RNAs during leaf development. J Exp Bot. 61(5):1277-91. doi: 10.1093/jxb/erp397.

34. Sunkar R, Li YF, Jagadeeswaran G. (2012) Functions of microRNAs in plant stress responses. Trends Plant Sci. 17(4):196-203. doi: 10.1016/j.tplants.2012.01.010.

35. Zhang L, Hou D, Chen X, Li D, Zhu L, Zhang Y, Li J, Bian Z, Liang X, Cai X, Yin Y, Wang C, Zhang T, Zhu D, Zhang D, Xu J, Chen Q, Ba Y, Liu J, Wang Q, Chen J, Wang J, Wang M, Zhang Q, Zhang J, Zen K, Zhang CY. (2012) Exogenous plant MIR168a specifically targets mammalian LDLRAP1: evidence of cross-kingdom regulation by microRNA. Cell Res. 22(1):107-26. doi: 10.1038/cr.2011.158.

36. Dickinson B, Zhang Y, Petrick JS, Heck G, Ivashuta S, Marshall WS. (2013) Lack of detectable oral bioavailability of plant microRNAs after feeding in mice. Nat Biotechnol. 31(11):965-7. doi: 10.1038/nbt.2737.

37. Snow JW, Hale AE, Isaacs SK, Baggish AL, Chan SY. Ineffective delivery of diet-derived microRNAs to recipient animal organisms. RNA Biol. 10(7):1107-16. doi: 10.4161/rna.24909

38. Liang G, Zhu Y, Sun B, Shao Y, Jing A, Wang J, Xiao Z. (2014) Assessing the survival of exogenous plant microRNA in mice. Food SciNutr. 2(4):380-8. doi: 10.1002/fsn3.113.

39. Chin AR, Fong MY, Somlo G, Wu J, Swiderski P, Wu X, Wang SE (2016) Cross-kingdom inhibition of breast cancer growth by plant miR159. Cell Res.; 26(2):217-28. doi: 10.1038/cr.2016.13.

40. Yanik H, Turktas M, Dundar E, Hernandez P, Dorado G, Unver T. (2013) Genome-wide identification of alternate bearing-associated microRNAs (miRNAs) in olive (Olea europaea L.) BMC Plant Biol.13:10. doi: 10.1186/1471-2229-13-10.

41. Pantaleo V, Szittya G, Moxon S, Miozzi L, Moulton V, Dalmay T, Burgyan (2010) Identification of grapevine microRNAs and their targets using high-throughput sequencing and degradome analysis. J. Plant J.62(6):960-76. doi: 10.1111/j.0960-7412.2010.04208.x.

42. Allen E, Xie Z, Gustafson AM, Sung GH, Spatafora JW, Carrington JC. (2004) Evolution of microRNA genes by inverted duplication of target gene sequences in Arabidopsis thaliana. Nat Genet. 36(12):1282-90.

43. Donaire L, Pedrola L, Rosa Rde L, Llave C. (2011) High-throughput sequencing of RNA silencing-associated small RNAs in olive (Olea europaea L.). PLoS One. 6(11):e27916. doi: 10.1371/journal.pone.0027916.

44. John B, Enright AJ, Aravin A, Tuschl T, Sander C, Marks DS. (2004) Human MicroRNA targets. PLoS Biol. 2(11).

45. Lewis BP, Burge CB, Bartel DP. (2005) Conserved seed pairing, often flanked by adenosines, indicates that thousands of human genes are microRNA targets. Cell. 120(1):15-20. doi: 10.1016/j.cell.2004.12.035.

46. Kertesz M, Iovino N, Unnerstall U, Gaul U, Segal E. (2007) The role of site accessibility in microRNA target recognition. Nature genetics. 39(10):1278-84. doi: 10.1038/ng2135.

47. Krek A, Grün D, Poy MN, Wolf R, Rosenberg L, Epstein EJ, MacMenamin P, da Piedade I, Gunsalus KC, Stoflel M, Rajewsky N. (2005) Combinatorial microRNA target predictions. Nat Genet. 37(5):495-500.

48. Coronnello C, Benos PV. (2013) ComiR: Combinatorial microRNA target prediction tool. Nucleic Acids Res. 41(Web Server issue):W159-64. doi: 10.1093/nar/gkt379.

49. Pirrò S, Zanella L, Kenzo M, Montesano C, Minutolo A, Potestà M, Sobze MS, Canini A, Cirilli M, Muleo R, Colizzi V, Galgani A. (2016) MicroRNA from Moringa oleifera: Identification by High Throughput Sequencing and Their Potential Contribution to Plant Medicinal Value. PLoS One. 11(3):e0149495.

50. Shahzad U, Khan M, Jaskani M, Khan I, Korban S. (2013) Genetic diversity and population structure of Moringa oleifera. Conservation Genetics. 14(6):1161-1172.

51. Anwar F, Latif S, Ashraf M, Gilani AH. (2007) Moringa oleifera: a food plant with multiple medicinal uses. Phytother Res. 21(1):17-25.

52. Abdull Razis AF, Ibrahim MD, Kntayya SB. (2014) Health benefits or Moringa oleifera.Asian Pac J Cancer Prev. 15(20):8571-8576.

53. Gismondi A, Canuti L, Impei S, Di Marco G, Kenzo M, Colizzi V (2013) Antioxidant extracts of African medicinal plants induce cell cycle arrest and differentiation in B16F10 melanoma cells. Int J Oncol. 43(3):956-964.

54. Jung IL. (2014) Soluble extract from Moringa oleifera leaves with a new anticancer activity. PLoS One. 9(4):e95492.

55. Chrupek M, Siipi H, Martinelli L. (2012) Bio-objects as "boundary crawlers:" the case of microRNAs. Croat Med J. 53(3):285-8.

56. Wang B. (2013) Base Composition Characteristics of Mammalian miRNAs. J Nucleic Acids. 2013:951570. doi: 10.1155/2013/951570.

57. Vergoulis T, Vlachos IS, Alexiou P, Georgakilas G, Maragkakis M, Reczko M, Gerangelos S, Koziris N, Dalamagas T, Hatzigeorgiou AG. (2012) TarBase 6.0: capturing the exponential growth of miRNA targets with experimental support. Nucleic acids research. 40(Database issue):D222-9. Epub 2011/12/03. doi: 10.1093/nar/gkr1161.

58. Bustin SA, Benes V, Garson JA, Hellemans J, Huggett J, Kubista M, Mueller R, Nolan T, Pfaffl MW, Shipley GL, Vandesompele J, Wittwer CT. (2009) The MIQE guidelines: minimum information for publication of quantitative real-time PCR experiments. Clin Chem. 55(4):611-22. doi: 10.1373/clinchem.2008.112797.

59. Kozomara A, Griffiths-Jones S. (2011) miRBase: integrating microRNA annotation and deep-sequencing data. Nucleic acids research. 39(Database issue):D152-7. Epub 2010/11/03. doi: 10.1093/nar/gkq1027.

60. Craig VJ, Tzankov A, Flori M, Schmid CA, Bader AG, Müller A. (2012) Systemic microRNA-34a delivery induces apoptosis and abrogates growth of diffuse large B-cell lymphoma in vivo. Leukemia. 26(13):2421-4. doi: 10.1038/leu.2012.110. 37.

61. Li XJ, Ren ZJ2, Tang JH1.(2014) MicroRNA-34a: a potential therapeutic target in human cancer.Cell Death Dis. 5:e1327. doi: 10.1038/cddis.2014.270.

## Patent documents cited in the description

1. US 20090227533 A1 Andreas G. Bader, Lubna Patrawala, Jason F. Wiggins, Mike W. Byrom, Charles D. Johnson, David Brown miR-34 Regulated Genes and Pathways as Targets for Therapeutic Intervention publ. date 10 Sep 2009

2. WO 2008137862 A2, Rosetta Inpharmatics Llc, Cold Spring Harbor Lab, Methods of using mir34 as a biomarker for tp53 functional status, publ. date 13 Nov, 2008

3. WO 2014209970 A1, Mirna Therapeutics, Inc., Biomarkers of mir-34 activity publ. date 31 Dec, 2014

4. WO 2009058766 A2, Government Of The U S A As Rep, Methods of regulating the expression of mir-34a and p18ink4c publ. date 7 May 2009

5. US8586727 B2, Mirna Therapeutics, Inc. Synthetic mimics of miR-34, publ. date 19 Nov 2013

6. US 8933051 B2, University Of Zurich, Treatment of B-cell lymphoma with microRNA, publ. date 13 Jan 2015

7. WO 2014181344 A2, Munisekhar Medasani, Satyasayee Babu Divi, Bioactive apolar extract containing plant genetic material for treatment of mammalian diseases publ. date13 Nov 2014

8. WO 2015026249 A1, Instytut Biochemii I Biofizyki Pan, Uniwersytet Im. Adama Mickiewicza W Poznaniu, Use of a mir172 molecule for decreasing inflammation, publ. date 26 Feb 2015

9. 20150291962, Chenyu Zhang, Junfeng Zhang, Ke Zeng, Lei Dong Extraction, preparation, and application of plant micro-ribonucleic acid, publ. date 15 Oct 2015

10. US 20090048187 A1, Luigi Ricciardiello, Clement Richard Boland, Marco Romano, Vincenzo Fogliano, Chemopreventive, Anticancer and Anti-Inflammatory Effects of Pinoresinol-Rich Olives, publ. date 19 Feb 2009

11. WO 2013162484 A1, Eraslan Mustafa, Tekin Mustafa, A combination of fig (ficus carica) fruit extract and olive (olea europaea) leaf extract publ. date 31 Oct 2013

12. US 20060222682 A1 David Andrews Nutraceutical Moringa composition publ. date 5 Oct 2006

13. CN 104224862 A Korea Atomic Energy Research Institute Composition containing moringa oleifera leave water soluble extract as effective component and used for treating or preventing cancer and preparation method for moringa oleifera leave extract publ. date 24 Dec 2014

SEQUENCE LISTING

SEQ. ID No: 1
plant-miR20

LENGTH: 23 nt
TYPE: nucleic acid
STRANDEDNESS: single
TOPOLOGY: linear
MOLECULAR TYPE: RNA molecule
SEQUENCE: ACAGUGGUGGUGGUGGUGGUGGU

SEQ. ID No: 1
**plant-miR20**

LENGTH: 23 nt
TYPE: nucleic acid
STRANDEDNESS: single
TOPOLOGY: linear
MOLECULAR TYPE: RNA molecule
SEQUENCE: ACAGUGGUGGUGGUGGUGGUGGU

SEQ. ID No. 2
**plant-miR27**

LENGTH: 22 nt
TYPE: nucleic acid
STRANDEDNESS: single
TOPOLOGY: linear
MOLECULAR TYPE: RNA molecule
SEQUENCE: UGGUGGCGGUGGCGGUGGCGGU

SEQ. ID No. 3
**plant-miR34**

LENGTH: 21 nt
TYPE: nucleic acid
STRANDEDNESS: single
TOPOLOGY: linear
MOLECULAR TYPE: RNA molecule
SEQUENCE: GGUGGCGGUGGAGGUGGAGGU

SEQ. ID No. 4
**plant-miR168a**

LENGTH: 21 nt
TYPE: nucleic acid
STRANDEDNESS: single
TOPOLOGY: linear
MOLECULAR TYPE: RNA molecule
SEQUENCE: UCGCUUGGUGCAGGUCGGGAC

**Claims**

1. Composition comprising plant-derived miRNA molecule *plant-miR-20* or its synthetic equivalents for use in the therapy of leukemia, wherein *plant-miR20* is represented by SEQ. ID No. 1.

2. Composition for use according to claim 1, wherein the leukemia is chosen from the group consisting of lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia and hairy cell leukemia.

3. Composition for use according to anyone of claims 1-2, wherein plant miR-20 reduces the level of non plant target gene encoding mammalian SIRT-1, BCL-2 protein expression, and proteins and oncoproteins of associated pathways (Bcl2/BAX balance).

4. Composition for use according to anyone of claims 1-3, wherein a plant-derived miRNA molecule or its synthetic equivalent reduces proliferation of cells according to claim 2.

**5.** Composition for use according to anyone of claims 1-4, wherein plant miR-20 has 2'- or 3'-0-methylation of the ribose of last nucleotide at the 3' end.

**6.** Composition for use according to anyone of claims 1-5, **characterized in that** the plant-derived miRNA molecule is provided from medicinal plants, fruit and vegetable plants, and ornamental plants, of which: *Abies alba, Abutilon theophrasti, Acanthus mollis, Acer campestre, Achillea spp, Aconitum napellus, Acorus calamus, Actaea spicata, Adiantum capillus veneris, Aesculus hippocastunum, Agave americana, Agrimonia eupatoria, Agropyrum repens, Agrostemma githago, Ailanthus altissima, Ajuga reptans, Alchemilla vulgaris, Alchemmila alpina, Alkanna tinctoria, Alliaria petiolata, Allium cepa, Allium porrum, Allium sativum L., Aloe spp, Althaea officinalis, Amaranthus retroflexus, Ambrosia artemisiifolia, Ammi majus, Amorphophallus konjac, Anacardium occidentaliss, Anagallis arvensis, Ananas sativus, Anchusa officinalis, Anemone nemorosa, Anethum graveolens, Angelica archangelica, Angelica sylvestris, Annona muricata, Antennaria dioica, Anthriscus cerefolium, Aquilegia vulgaris, Arbutus unedo, Arctium lappa L, Arctostaphylos uva-ursi, Aristolochia clematitis, Aristotelia chilensis, Arnica montan, Artemisia absinthium, Artemisia dranunculus, Artemisia vulgaris, Asarum europaeum, Asparagus officinalis, Asperula odorata, Aster amellus, Atriplex hortensis, Aucuba Japonica, Ballota nigra, Balsamita major, Barbarea vulgaris, Bellis perennis, Berberis vulgaris, Betula pendula, Bixa orellana, βorago officinalis, Boswellia serrata, Bryonia dioica, Buddleja davidii, Buxus sempervirens, Calamintha nepeta, Calendula arvensis, Calendula officinalis, Calluna vulgaris, Caltha palustris, Camellia sinensis, Campanula rapunculus, Cananga odorata, Cannabis sativa, Capparis spinosa, Capsella bursa pastoris, Capsicum spp, Cardamine bulbifera, Cardamine hirsuta, Carlina acaulis, Carthamus tinctorius, Carum carvi, Castanea sativa, Cedrus atlantica, Centaurea cyanus, Centaurium erythraea, Centella asiatica, Ceratonia siliqua, Cercis siliquastrum, Cetraria islandica, Chamomilla matricaria, Chelidonium majus, Chenopodium album, Chrysanthemum cinerariaefolium, Cichorium intybus, Cinchona officinalis, Cirsium arvense, Cirsium vulgare, Cistus monspeliensis, Citrus limon, Clematis vitalba, Clerodendrum trichòtomum, Clinopodium vulgare, Cnicus benedictus, Cochlearia officinalis, Cola acuminate, Conium maculatum, Convolvulus arvensis, Coriandrum sativum, Corylus avellana, Crataegus monogyna, Crepis vesicaria. Crocus biflorus, Crocus sativus, Croton lechleris, Ciuciata laevipes, Cucurbita pepo, Cyclanthera pedata, Cymbalaria muralis, Cymbopogon spp, Cynara scolymus, Cynodon dactylon, Cytisus scoparius, Datura stramonium, Daucus carota, Descurainia sophia, Dictamnus albus, Diplotaxis tenuifolia, Dipsacus fullonum, Drosera rotundifoli, Dryopteris felix-mas, Duchesnea indica, Echinacea pallida, Echinacea purpurea, Echinops ritro, Echium vulgare, Elutherococcus senticosus, Epilobium hirsutum, Equisetum arvense, Erica carnea, Erigeron canadensis, Eriobotrya japonica, Erodium cicutarium, Eruca sativa, Eryngium campestre, Eschscholtzia californica, Eucalyptus globulus, Eugenia caryophyllata, Eupatorium cannabinum, Euphorbia lathyris, Euterpe oleracea, Fagus sylvatica, Ficus carica, Filipendula ulmaria, Foeniculum vulgare Miller subsp. vulgare var. dulce, Fragola selvatica, Frangula alnus, Fraxinus excelsior, Fucus vesiculosus, Fumaria officinalis, Galega officinalis, Galeopsis pubescens, Galeopsis tetrahit, Galinsoga parviflora, Galium aparine, Galium verum, Garcinia cambogia, Gentiana lutea, Geranium dissectum, Geranium molle, Geum urbanum, Ginkgo biloba, Glechoma hederacea, Globularia punctata, Glycyrrhiza glabra, Gra1iola officinalis, Grindelia robusta, Gymnema sylvestris, Hamamelis virginiana, Harpagophytum procumbens, Hedera helix, Helianthus annuus, Helianthus tuberosus, Helichrysum italicum, Herniaria glabra, Hibiscus syriacus, Hieracium pilosella, Hippophae rhamnoides, Humulus lupulus, Hydrangea macrophylla, Hyoscyamus niger, Hypericum perforatum, Hypochoeris radicata, Hyssopus officinalis, Ilex aquifolium, Illicium verum, Impatiens glandulifera, Impatiens noli-langere, Inula helenium, Iris pseudacorus, Isatis tinctoria, Jacobaea vulgaris, Jasminum officinale, Juglans regia, Juniperus communis, Juniperus oxycedrus, Lactuca serriola, Lamium album, Lamium amplexicaule, Lamium galeobdolon, Lamium maculatum, Lamium purpureum, Lapsana communis, Laurus nobilis, Lavandula spp, Leontopodium alpinum, Leonurus cardiac, Lepidium virginicum, Leucanthemum vulgare, Levisticum officinale, Liatris spicata, Linaria vulgaris, Linum usitatissimum, Lippia citriodora, Lithospermum officinale, Lobularia maritime, Lonicera caprifolium, Lonicera japonica, Lotus corniculatus, Lunaria annua, Lycium barbarum, Lycopus europaeus, Lysimachia vulgaris, Lythrum salicaria, Mahonia aquifolium, Malus domestica, Malva alcea, Malva silvestris, Matricaria chamomilla, Medicago sativa, Melilotus officinalis, Melissa officinalis, Melittis melissophyllum, Mentha spp, Menyanthes trifoliate, Mespilus germanica, Mirabilis jalapa, Moringa oleifera, Morus alba, Myosotis sylvatica, Myrrhis odorata, Myrtus communis, Narcissus tazetta, Nasturtium officinale, Nelumbo nucifera, Nepeta cataria, Nicotiana glauca, Nicotiana tabacum, Nigella sativa, Nuphar lutea, Nymphaea alba, Ocimum basilicum, Oenanthe aquatic, Oenothera biennis, Olea europaea, Ononis spinosa, Origanum majorana, Origanum vulgare, Ornithogalum umbeilatum, Oxalis acetosella, Oxalis pes-caprae, Paeonia officinalis, Panax ginseng, Panicum miliaceum, Papaver rhoeas, Papaver somniferum, Parieturia officinalis, Purthenocissus quinquefolia, Passiflora incarnate, Pastinaca sativa, Paulinia sorbilis, Petroselinum spp, Peucedanum ostruthium, Peumus boldus, Phragmites australis, Physalis alkekengi, Phytolacca decandra, Picea excelsa, Pimpinella anisum, Pinus mugo, Pinus sylvestris, Piper nigrum, Plantago lanceolata, Plantago Major, Plantago psyllium, Polygala vulgaris, Polygonum aviculare, Polygonum bistorta, Polygonum hydropiper, Portulaca oleraceae, Potentilla reptans, Primula spp, Prunella vulgaris, Primus spinosa, Pulmonaria officinalis, Punica granatum, Quercus ilex, Quercus palustris, Quercus robur,*

*Quercus rubra, Ranunculus bulbosus, Ranunculus ficaria, Ranunculus sceleratus, Raphanus raphanistrum, Rhodiola rosea, Ribes nigrum, Ribes rubrum, Ricinus communis, Robinia pseudoacacia, Rosa canina, Rosmarinus officinalis, Rubus idaeus, Rubus ulmifolius, Rumex acetosa, Rumex acetosella, Rumex conglomerates, Rumex crispus, Rumex obtusifolius, Ruscus aculeatus, Ruta graveolens, Saccharomyces cerevisiae, Salix alba, Salix babylonica, Salix cinerea, Salvia pratensis, Salvia spp, Sambucus nigra, Sanicula europaea, Saponaria ocymoides, Saponaria officinalis, Salureja spp, Scilla bifolia, Scrophularia nodosa. Scutellaria galericulata, Sedum maximum, Sempervivum tectorum, Senecio vulgaris, Silybum marianum, Sinapis spp, Smilax aspera, Solarium dulcamara, Solanum melongena, Solanum nigrum, Sonchus asper, Sorbus aucuparia, Sorbus domestica, Stachys palustris, Stellaria aquatica, Stellaria media, Symphytum officinalis, Syringa vulgaris, Tanacetum vulgare, Taraxacum officinale, Taxus baccata, Tecoma impetiginosa, Teucrium chamaedrys, Theobroma cacao, Thuja occidentalis, Thymus spp, Tilia platyphyllos, Tilia spp, Tragopogon pratensis, Trifolium pretense, Trifolium repens, Trigonella foenum-graecum, Triticum aestivum, Tropaeolum majus, Tussilago farfara, Typha latifolia, Ulmus minor, Umbilicus rupestris, Uncaria tomentosa, Urtica dioica, Urtica spp, Vaccinium myrtillus, Valeriana officinalis, Valerianella locusta, Verbascum sinuatum, Verhascum Thapsus, Verbena officinalis, Veronica anagallis-aquatica, Veronica persica, Vinca minor, Viola odorata, Viola tricolor, Vitex agnus-castus, Xanthium strumarium, Zea mays, Zingiber officinalis, Ziziphus jujube.*

7. Composition for use according to anyone of claims 1-6, wherein the composition is provided as the water-soluble and/or liposoluble extracts of plants.

8. Composition for use according to anyone of claims 1-7, wherein the composition is administered orally containing one or more pharmaceutically acceptable carriers.

9. Composition for use according to anyone of claims 1-8, wherein the pharmaceutically acceptable carrier further comprises one or more selected from the group consisting of water, saline, starches, sugars, gels, lipids, waxes, glycerol, solvents, oils, liquids, proteins, glycols, electrolyte solutions, alcohols, fillers, binders, emulsifiers, humectants, preservatives, buffers, colorants, emollients, foaming agents, sweeteners, thickeners, surfactants, additives and solvents and mixtures thereof.

10. Composition for use according to anyone of the claims 1-8, wherein the composition is provided as pasta or granular preparations for vegetable soup.

**Patentansprüche**

1. Zusammensetzung, die das miRNA-Molekül pflanzlichen Ursprungs - plant-miR-20 oder seine synthetischen Äquivalente - zur Verwendung in der Leukämietherapie enthält, in der das plant-miR20-Molekül durch die SEQ. ID Nr. 1 dargestellt ist.

2. Zusammensetzung zur Verwendung nach Patentanspruch 1, wobei die Art der Leukämie aus der Gruppe der lymphatischen Leukämien, plasmazellulären Leukämien, Erythroleukämien, lymphosarkomatischen Zellleukämien, myelogenen Leukämien, mastzellulären Leukämien, eosinophilen Leukämien, monozytischen Leukämien, mastozytären Leukämien, megakaryoblastischen Leukämien und Haarzell-Leukämien gewählt ist.

3. Zusammensetzung zur Verwendung nach einem der Patentansprüche 1-2, bei der das plant-miR-20-Molekül den Gehalt des nicht pflanzlichen Zielgens, das für die Expression der Säugetierproteine SIRT-1 und BCL-2 kodiert, sowie der zugehörigen Proteine und Onkoproteine reduziert (Rest BCL-2/BAX).

4. Zusammensetzung zur Verwendung nach einem der Patentansprüche 1-3, wobei das plant-miRNA-Molekül oder seine synthetischen Äquivalente die Zellproliferation nach Patentanspruch 2 reduzieren.

5. Zusammensetzung zur Verwendung nach einem der Patentansprüche 1-4, in der das plant- miR-20-Molekül eine 2'- oder 3'-0-Methylierung der Ribose des letzten Nukleotids am 3'- Ende aufweist.

6. Zusammensetzung zur Verwendung nach einem der Patentansprüche 1-5, wobei das plant-miRNA-Molekül von Arzneipflanzen, Obst, Gemüse sowie Zierpflanzen stammt, wie: *Abies alba, Abutilon theophrasti, Acanthus mollis, Acer campestre, Achillea spp, Aconitum napellus, Acorus calamus, Actaea spicata, Adiantum capillus veneris, Aesculus hippocastanum, Agave americana, Agrimonia eupatoria, Agropyrum repens, Agrostemma githago, Ailanthus altissima, Ajuga reptans, Alchemilla vulgaris, Alchemmila alpina, Alkanna tinctoria, Alliaria petiolata, Allium*

*cepa, Allium porrum, Allium sativum L., Aloe spp, Althaea officinalis, Amaranthus retroflexus, Ambrosia artemisiifolia, Ammi majus, Amorphophallus konjac, Anacardium occidentaliss, Anagallis arvensis, Ananas sativus , Anchusa officinalis, Anemone nemorosa, Anethum graveolens, Angelica archangelica, Angelica sylvestris, Annona muricata, Antennaria dioica, Anthriscus cerefolium, Aquilegia vulgaris, Arbutus unedo, Arctium lappa L, Arctostaphylos uva-ursi, Aristolochia clematitis, Aristotelia chilensis, Arnica montan, Artemisia absinthium, Artemisia dranunculus, Artemisia vulgaris, Asarum europaeum, Asparagus officinalis, Asperula odorata, Aster amellus, Atriplex hortensis, Aucuba Japonica, Ballota nigra, Balsamita major, Barbarea vulgaris, Bellis perennis, Berberis vulgaris, Betula pendula, Bixa orellana, Borago officinalis, Boswellia serrata, Bryonia dioica, Buddleja davidii, Buxus sempervirens, Calamintha nepeta, Calendula arvensis, Calendula officinalis, Calluna vulgaris, Caltha palustris, Camellia sinensis, Campanula rapunculus, Cananga odorata, Cannabis sativa, Capparis spinosa, Capsella bursa pastoris, Capsicum spp, Cardamine bulbifera, Cardamine hirsuta, Carlina acaulis, Carthamus tinctorius, Carum carvi, Castanea sativa, Cedrus atlantica, Centaurea cyanus, Centaurium erythraea, Centella asiatica, Ceratonia siliqua, Cercis siliquastrum, Cetraria islandica, Chamomilla matricaria, Chelidonium majus, Chenopodium album, Chrysanthemum cinerariaefolium, Cichorium intybus, Cinchona officinalis, Cirsium arvense, Cirsium vulgare, Cistus monspeliensis, Citrus limon, Clematis vitalba, Clerodendrum trichòtomum, Clinopodium vulgare, Cnicus benedictus, Cochlearia officinalis, Cola acuminate, Conium maculatum, Convolvulus arvensis, Coriandrum sativum, Corylus avellana, Crataegus monogyna, Crepis vesicaria, Crocus biflorus, Crocus sativus, Croton lechleris, Cruciata laevipes, Cucurbita pepo, Cyclanthera pedata, Cymbalaria muralis, Cymbopogon spp, Cynara scolymus, Cynodon dactylon, Cytisus scoparius, Datura stramonium, Daucus carota, Descurainia sophia, Dictamnus albus, Diplotaxis tenuifolia, Dipsacus fullonum, Drosera rotundifoli, Dryopteris felix-mas, Duchesnea indica, Echinacea pallida, Echinacea purpurea, Echinops ritro, Echium vulgare, Elutherococcus senticosus, Epilobium hirsutum, Equisetum arvense, Erica carnea, Erigeron canadensis, Eriobotrya japonica, Erodium cicutarium, Eruca sativa, Eryngium campestre, Eschscholtzia californica, Eucalyptus globulus, Eugenia caryophyllata, Eupatorium cannabinum, Euphorbia lathyris, Euterpe oleracea, Fagus sylvatica, Ficus carica, Filipendula ulmaria, Foeniculum vulgare Miller subsp. vulgare var. dulce, Fragola selvatica, Frangula alnus, Fraxinus excelsior, Fucus vesiculosus, Fumaria officinalis, Galega officinalis, Galeopsis pubescens, Galeopsis tetrahit, Galinsoga parviflora, Galium aparine, Galium verum, Garcinia cambogia, Gentiana lutea, Geranium dissectum, Geranium molle, Geum urbanum, Ginkgo biloba, Glechoma hederacea, Globularia punctata, Glycyrrhiza glabra, Gratiola officinalis, Grindelia robusta, Gymnema sylvestris, Hamamelis virginiana, Harpagophytum procumbens, Hedera helix, Helianthus annuus, Helianthus tuberosus, Helichrysum italicum, Herniaria glabra, Hibiscus syriacus, Hieracium pilosella, Hippophae rhamnoides, Humulus lupulus, Hydrangea macrophylla, Hyoscyamus niger, Hypericum perforatum, Hypochoeris radicata, Hyssopus officinalis, Ilex aquifolium, Illicium verum, Impatiens glandulifera, Impatiens noli-tangere, Inula helenium, Iris pseudacorus, Isatis tinctoria, Jacobaea vulgaris, Jasminum officinale, Juglans regia, Juniperus communis, Juniperus oxycedrus, Lactuca serriola, Lamium album, Lamium amplexicaule, Lamium galeobdolon, Lamium maculatum, Lamium purpureum, Lapsana communis, Laurus nobilis, Lavandula spp, Leontopodium alpinum, Leonurus cardiac, Lepidium virginicum, Leucanthemum vulgare, Levisticum officinale, Liatris spicata, Linaria vulgaris, Linum usitatissimum, Lippia citriodora, Lithospermum officinale, Lobularia maritime, Lonicera caprifolium, Lonicera japonica, Lotus corniculatus, Lunaria annua, Lycium barbarum, Lycopus europaeus, Lysimachia vulgaris, Lythrum salicaria, Mahonia aquifolium, Malus domestica, Malva alcea, Malva silvestris, Matricaria chamomilla, Medicago sativa, Melilotus officinalis, Melissa officinalis, Melittis melissophyllum, Mentha spp, Menyanthes trifoliate, Mespilus germanica, Mirabilis jalapa, Moringa oleifera, Morus alba, Myosotis sylvatica, Myrrhis odorata, Myrtus communis, Narcissus tazetta, Nasturtium officinale, Nelumbo nucifera, Nepeta cataria, Nicotiana glauca, Nicotiana tabacum, Nigella sativa, Nuphar lutea, Nymphaea alba, Ocimum basilicum, Oenanthe aquatic, Oenothera biennis, Olea europaea, Ononis spinosa, Origanum majorana, Origanum vulgare, Ornithogalum umbellatum, Oxalis acetosella, Oxalis pes-caprae, Paeonia officinalis, Panax ginseng, Panicum miliaceum, Papaver rhoeas, Papaver somniferum, Parietaria officinalis, Parthenocissus quinquefolia, Passiflora incarnate, Pastinaca sativa, Paulinia sorbilis, Petroselinum spp, Peucedanum ostruthium, Peumus boldus, Phragmites australis, Physalis alkekengi, Phytolacca decandra, Picea excelsa, Pimpinella anisum, Pinus mugo, Pinus sylvestris, Piper nigrum, Plantago lanceolata, Plantago Major, Plantago psyllium, Polygala vulgaris, Polygonum aviculare, Polygonum bistorta, Polygonum hydropiper, Portulaca oleraceae, Potentilla reptans, Primula spp, Prunella vulgaris, Prunus spinosa, Pulmonaria officinalis, Punica granatum, Quercus ilex, Quercus palustris, Quercus robur, Quercus rubra, Ranunculus bulbosus, Ranunculus ficaria, Ranunculus sceleratus, Raphanus raphanistrum, Rhodiola rosea, Ribes nigrum, Ribes rubrum, Ricinus communis, Robinia pseudoacacia, Rosa canina, Rosmarinus officinalis, Rubus idaeus, Rubus ulmifolius, Rumex acetosa, Rumex acetosella, Rumex conglomerates, Rumex crispus, Rumex obtusifolius, Ruscus aculeatus, Ruta graveolens, Saccharomyces cerevisiae, Salix alba, Salix babylonica, Salix cinerea, Salvia pratensis, Salvia spp, Sambucus nigra, Sanicula europaea, Saponaria ocymoides, Saponaria officinalis, Satureja spp, Scilla bifolia, Scrophularia nodosa, Scutellaria galericulata, Sedum maximum, Sempervivum tectorum, Senecio vulgaris, Silybum marianum, Sinapis spp, Smilax aspera, Solanum dulcamara, Solanum melongena, Solanum nigrum, Sonchus asper, Sorbus aucuparia, Sorbus domestica, Stachys palustris, Stellaria aquatica, Stellaria*

*media, Symphytum officinalis, Syringa vulgaris, Tanacetum vulgare, Taraxacum officinale, Taxus baccata, Tecoma impetiginosa, Teucrium chamaedrys, Theobroma cacao, Thuja occidentalis, Thymus spp, Tilia platyphyllos, Tilia spp, Tragopogon pratensis, Trifolium pretense, Trifolium repens, Trigonella foenum-graecum, Triticum aestivum, Tropaeolum majus, Tussilago farfara, Typha latifolia, Ulmus minor, Umbilicus rupestris, Uncaria tomentosa, Urtica dioica, Urtica spp, Vaccinium myrtillus, Valeriana officinalis, Valerianella locusta, Verbascum sinuatum, Verbascum Thapsus, Verbena officinalis, Veronica anagallis-aquatica, Veronica persica, Vinca minor, Viola odorata, Viola tricolor, Vitex agnus-castus, Xanthium strumarium, Zea mays, Zingiber officinalis, Ziziphus jujube.*

7. Zusammensetzung zur Verwendung nach einem der Patentansprüche 1-6, wenn diese Zusammensetzung in Wasser löslich ist oder in Form eines fettlöslichen Pflanzenextrakts formuliert ist.

8. Zusammensetzung zur Verwendung nach einem der Patentansprüche 1-7, wobei die Zusammensetzung oral zusammen mit einem oder mehreren akzeptablen pharmazeutischen Trägern verabreicht wird.

9. Zusammensetzung zur Verwendung nach einem der Patentansprüche 1-8, wobei der akzeptable pharmazeutische Träger zusätzlich oder mehr als die folgenden Träger beinhaltet: Wasser, Lösung, Stärke, Zucker, Gelatine, Lipide, Wachse, Glycerin, Lösungsmittel, Öle, Flüssigkeiten, Proteine, Glykole, Elektrolytlösungen, Alkohole, Füllstoffe, Dichtungsmittel, Emulgatoren, Emulgatoren, Feuchthaltemittel, Schutzmittel, Puffer, Farbstoffe, Weichmacher, Schäume, Süßungsmittel, Eindickmittel, Tenside, Zusatzstoffe und Lösungsmittel, auch in Mischung.

10. Zusammensetzung zur Verwendung nach einem der Patentansprüche 1-8, wobei die Zusammensetzung in Form von Paste oder granulierten Zubereitungen für Gemüsepüree vorliegt.

## Revendications

1. Composition qui comprend la molécule miRNA d'origine végétale - *plant-miR-20,* ou ses équivalents synthétiques, pour son utilisation dans le traitement des leucémies, où la molécule *plant-miR20* est représentée par la SEQ. ID N° 1.

2. Composition conforme à la revendication 1, où le type de leucémie est choisi dans le groupe des leucémies lymphoïdes, leucémies plasma cellulaires, érythroleucémies, leucémies à cellules lymphosarcomatiques; leucémies myélogéniques, leucémies à mastocytes, leucémies éosinophiles, leucémies monocytiques, leucémies mastocytaires, leucémies aiguës mègacaryoblastiques et leucemies à cellules chevelues.

3. Composition conforme à l'une des revendications 1-2 dans laquelle la molécule *plant miR-20* réduit le niveau du gène-cible non végétal qui codifie l'expression des protéines mammifères SIRT-1 et BCL-2, comme également des protéines et des oncoprotéines associées (équilibre BCL-2/BAX).

4. Composition conforme à l'une des revendications 1-3 dans laquelle la molécule végétale miRNA ou ses équivalents synthétiques réduisent la prolifération des cellules selon la revendication 2.

5. Composition conforme à l'une des revendications 1-4 où la molécule végétale miR-20 a 2'- ou 3'-0 méthylation du ribose du dernier nucléotide à l'extrémité 3'.

6. Composition conforme à l'une des revendications 1-5 dans laquelle la molécule végétale miRNA provient de plantes médicinales, fruits, légumes verts ou plantes ornementales, comme par exemple: *Abies alba, Abutilon theophrasti, Acanthus mollis, Acer campestre, Achillea spp, Aconitum napellus, Acorus calamus, Actaea spicata, Adiantum capillus veneris, Aesculus hippocastanum, Agave americana, Agrimonia eupatoria, Agropyrum repens, Agrostemma githago, Ailanthus altissima, Ajuga reptans, Alchemilla vulgaris, Alchemmila alpina, Alkanna tinctoria, Alliaria petiolata, Allium cepa, Allium porrum, Allium sativum L., Aloe spp, Althaea officinalis, Amaranthus retroflexus, Ambrosia artemisiifolia, Ammi majus, Amorphophallus konjac, Anacardium occidentaliss, Anagallis arvensis, Ananas sativus , Anchusa officinalis, Anemone nemorosa, Anethum graveolens, Angelica archangelica, Angelica sylvestris, Annona muricata, Antennaria dioica, Anthriscus cerefolium, Aquilegia vulgaris, Arbutus unedo, Arctium lappa L, Arctostaphylos uva-ursi, Aristolochia clematitis, Aristotelia chilensis, Arnica montan, Artemisia absinthium, Artemisia dranunculus, Artemisia vulgaris, Asarum europaeum, Asparagus officinalis, Asperula odorata, Aster amellus, Atriplex hortensis, Aucuba Japonica, Ballota nigra, Balsamita major, Barbarea vulgaris, Bellis perennis, Berberis vulgaris, Betula pendula, Bixa orellana, Borago officinalis, Boswellia serrata, Bryonia dioica, Buddleja davidii, Buxus sempervirens, Calamintha nepeta, Calendula arvensis, Calendula officinalis, Calluna vulgaris, Caltha palustris, Camellia*

*sinensis, Campanula rapunculus, Cananga odorata, Cannabis sativa, Capparis spinosa, Capsella bursa pastoris, Capsicum spp, Cardamine bulbifera, Cardamine hirsuta, Carlina acaulis, Carthamus tinctorius, Carum carvi, Castanea sativa, Cedrus atlantica, Centaurea cyanus, Centaurium erythraea, Centella asiatica, Ceratonia siliqua, Cercis siliquastrum, Cetraria islandica, Chamomilla matricaria, Chelidonium majus, Chenopodium album, Chrysanthemum cinerariaefolium, Cichorium intybus, Cinchona officinalis, Cirsium arvense, Cirsium vulgare, Cistus monspeliensis, Citrus limon, Clematis vitalba, Clerodendrum trichòtomum, Clinopodium vulgare, Cnicus benedictus, Cochlearia officinalis, Cola acuminate, Conium maculatum, Convolvulus arvensis, Coriandrum sativum, Corylus avellana, Crataegus monogyna, Crepis vesicaria, Crocus biflorus, Crocus sativus, Croton lechleris, Cruciata laevipes, Cucurbita pepo, Cyclanthera pedata, Cymbalaria muralis, Cymbopogon spp, Cynara scolymus, Cynodon dactylon, Cytisus scoparius, Datura stramonium, Daucus carota, Descurainia sophia, Dictamnus albus, Diplotaxis tenuifolia, Dipsacus fullonum, Drosera rotundifoli, Dryopteris felix-mas, Duchesnea indica, Echinacea pallida, Echinacea purpurea, Echinops ritro, Echium vulgare, Elutherococcus senticosus, Epilobium hirsutum, Equisetum arvense, Erica carnea, Erigeron canadensis, Eriobotrya japonica, Erodium cicutarium, Eruca sativa, Eryngium campestre, Eschscholtzia californica, Eucalyptus globulus, Eugenia caryophyllata, Eupatorium cannabinum, Euphorbia lathyris, Euterpe oleracea, Fagus sylvatica, Ficus carica, Filipendula ulmaria, Foeniculum vulgare Miller subsp. vulgare var. dulce, Fragola selvatica, Frangula alnus, Fraxinus excelsior, Fucus vesiculosus, Fumaria officinalis, Galega officinalis, Galeopsis pubescens, Galeopsis tetrahit, Galinsoga parviflora, Galium aparine, Galium verum, Garcinia cambogia, Gentiana lutea, Geranium dissectum, Geranium molle, Geum urbanum, Ginkgo biloba, Glechoma hederacea, Globularia punctata, Glycyrrhiza glabra, Gratiola officinalis, Grindelia robusta, Gymnema sylvestris, Hamamelis virginiana, Harpagophytum procumbens, Hedera helix, Helianthus annuus, Helianthus tuberosus, Helichrysum italicum, Herniaria glabra, Hibiscus syriacus, Hieracium pilosella, Hippophae rhamnoides, Humulus lupulus, Hydrangea macrophylla, Hyoscyamus niger, Hypericum perforatum, Hypochoeris radicata, Hyssopus officinalis, Ilex aquifolium, Illicium verum, Impatiens glandulifera, Impatiens noli-tangere, Inula helenium, Iris pseudacorus, Isatis tinctoria, Jacobaea vulgaris, Jasminum officinale, Juglans regia, Juniperus communis, Juniperus oxycedrus, Lactuca serriola, Lamium album, Lamium amplexicaule, Lamium galeobdolon, Lamium maculatum, Lamium purpureum, Lapsana communis, Laurus nobilis, Lavandula spp, Leontopodium alpinum, Leonurus cardiac, Lepidium virginicum, Leucanthemum vulgare, Levisticum officinale, Liatris spicata, Linaria vulgaris, Linum usitatissimum, Lippia citriodora, Lithospermum officinale, Lobularia maritime, Lonicera caprifolium, Lonicera japonica, Lotus corniculatus, Lunaria annua, Lycium barbarum, Lycopus europaeus, Lysimachia vulgaris, Lythrum salicaria, Mahonia aquifolium, Malus domestica, Malva alcea, Malva silvestris, Matricaria chamomilla, Medicago sativa, Melilotus officinalis, Melissa officinalis, Melittis melissophyllum, Mentha spp, Menyanthes trifoliate, Mespilus germanica, Mirabilis jalapa, Moringa oleifera, Morus alba, Myosotis sylvatica, Myrrhis odorata, Myrtus communis, Narcissus tazetta, Nasturtium officinale, Nelumbo nucifera, Nepeta cataria, Nicotiana glauca, Nicotiana tabacum, Nigella sativa, Nuphar lutea, Nymphaea alba, Ocimum basilicum, Oenanthe aquatic, Oenothera biennis, Olea europaea, Ononis spinosa, Origanum majorana, Origanum vulgare, Ornithogalum umbellatum, Oxalis acetosella, Oxalis pes-caprae, Paeonia officinalis, Panax ginseng, Panicum miliaceum, Papaver rhoeas, Papaver somniferum, Parietaria officinalis, Parthenocissus quinquefolia, Passiflora incarnate, Pastinaca sativa, Paulinia sorbilis, Petroselinum spp, Peucedanum ostruthium, Peumus boldus, Phragmites australis, Physalis alkekengi, Phytolacca decandra, Picea excelsa, Pimpinella anisum, Pinus mugo, Pinus sylvestris, Piper nigrum, Plantago lanceolata, Plantago Major, Plantago psyllium, Polygala vulgaris, Polygonum aviculare, Polygonum bistorta, Polygonum hydropiper, Portulaca oleraceae, Potentilla reptans, Primula spp, Prunella vulgaris, Prunus spinosa, Pulmonaria officinalis, Punica granatum, Quercus ilex, Quercus palustris, Quercus robur, Quercus rubra, Ranunculus bulbosus, Ranunculus ficaria, Ranunculus sceleratus, Raphanus raphanistrum, Rhodiola rosea, Ribes nigrum, Ribes rubrum, Ricinus communis, Robinia pseudoacacia, Rosa canina, Rosmarinus officinalis, Rubus idaeus, Rubus ulmifolius, Rumex acetosa, Rumex acetosella, Rumex conglomerates, Rumex crispus, Rumex obtusifolius, Ruscus aculeatus, Ruta graveolens, Saccharomyces cerevisiae, Salix alba, Salix babylonica, Salix cinerea, Salvia pratensis, Salvia spp, Sambucus nigra, Sanicula europaea, Saponaria ocymoides, Saponaria officinalis, Satureja spp, Scilla bifolia, Scrophularia nodosa, Scutellaria galericulata, Sedum maximum, Sempervivum tectorum, Senecio vulgaris, Silybum marianum, Sinapis spp, Smilax aspera, Solanum dulcamara, Solanum melongena, Solanum nigrum, Sonchus asper, Sorbus aucuparia, Sorbus domestica, Stachys palustris, Stellaria aquatica, Stellaria media, Symphytum officinalis, Syringa vulgaris, Tanacetum vulgare, Taraxacum officinale, Taxus baccata, Tecoma impetiginosa, Teucrium chamaedrys, Theobroma cacao, Thuja occidentalis, Thymus spp, Tilia platyphyllos, Tilia spp, Tragopogon pratensis, Trifolium pretense, Trifolium repens, Trigonella foenum-graecum, Triticum aestivum, Tropaeolum majus, Tussilago farfara, Typha latifolia, Ulmus minor, Umbilicus rupestris, Uncaria tomentosa, Urtica dioica, Urtica spp, Vaccinium myrtillus, Valeriana officinalis, Valerianella locusta, Verbascum sinuatum, Verbascum Thapsus, Verbena officinalis, Veronica anagallis-aquatica, Veronica persica, Vinca minor, Viola odorata, Viola tricolor, Vitex agnus-castus, Xanthium strumarium, Zea mays, Zingiber officinalis, Ziziphus jujube.*

7. Composition conforme à l'une des revendications 1-6 du fait que cette Composition est soluble dans l'eau, ou formulée sous forme d'extrait liposoluble de plantes.

8. Composition conforme à l'une des 1-7 du fait que cette Composition est administrée oralement avec un ou plusieurs véhicules pharmacologiquement admissibles.

9. Composition conforme à l'une des revendications 1-8 du fait que le véhicule pharmacologiquement admissible comprend également, ou en plus, les véhicules suivants: eau, solution, amides ou fécules, sucres, gélatines, lipides, cires, glycérol, solvants, huiles, liquides, protéines, glycols, solutions électrolythiques, alcool, remplissages, scellants, émulsionnants, humidifiants, protecteurs, tampons, colorants, émollients, mousses, édulcorants, épaississeurs tensioactifs, additifs ou solvants, éventuellement en mélange.

10. Composition conforme à l'une des revendications 1-8 du fait que la Composition se présente sous forme de pâte ou de granulats pour potages de légumes.

# Fig. 1

**A)**

| 172 Plant miRNAs | 2042 Animal miRNAs |

Comparison Phase

Sliding between plant and animal miRNAs with a movement window = 1

Selection of the best matching percentage

351,224 Total comparisons

Filtering Phase

FALSE → 339,090 Discarded

TRUE

12,134 accepted

FALSE → 9,970 Discarded

TRUE

2,164 full-filtered comparisons:

+ 117 *plant* microRNAs

+ 1,001 *H. sapiens* microRNAs

**B)**

```
5' UGG CAG UGU CUU AGC UGG UUG U    hsa-miR34a
    | | |   |  | |       |   | | | |
5' UGG UGG UGG UGG UGG UGG UGA CA    plant-miR20
```

# Fig. 2

**A)**

JURKAT (72 hours post transfection)

**B)**

# Fig. 3

A) ----- Untreated cells = 1

B)

C)

# Fig. 3

# Fig. 4

A)

B)

# Fig. 5

Fig. 6

## Fig. 7

-80°C 24 hours

M.oleifera mg/ml

0
0,01
0,1
0,25
0,5
1
2,5
5
10

A) Leaves

B) Immature seeds

C) Mature seeds

# Fig. 8

**Boiling extract**

# Fig. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20090227533 A1
- WO 2008137862 A2
- WO 2014209970 A1
- WO 2009058766 A2
- US 8586727 B2
- US 8933051 B2
- WO 2014181344 A2

- WO 2015026249 A1
- WO 20150291962 A
- US 20090048187 A1
- WO 2013162484 A1
- US 20060222682 A1
- CN 104224862 A

**Non-patent literature cited in the description**

- Remington's Pharmaceutical Sciences. Williams & Wilkins
- **BARTEL DP.** MicroRNAs: target recognition and regulatory functions. *Cell,* 2009, vol. 136 (2), 215-33
- **HUANG Y ; SHEN XJ ; ZOU Q ; ZHAO QL.** Biological functions of microRNAs. *BioorgKhim.,* 2010, vol. 36 (6), 747-52
- **LIU Q ; WANG F ; AXTELL MJ.** Analysis of complementarity requirements for plant microRNA targeting using a Nicotianabenthamiana quantitative transient assay. *The Plant cell,* 2014, vol. 26 (2), 741-53
- **MALLORY AC ; REINHART BJ ; JONES-RHOADES MW ; TANG G ; ZAMORE PD ; BARTON MK ; BARTEL DP.** MicroRNA control of PHABULOSA in leaf development: importance of pairing to the microRNA 5' region. *The EMBO journal,* 2004, vol. 23 (16), 3356-64
- **XIE M ; ZHANG S ; YU B.** microRNA biogenesis, degradation and activity in plants. *Cellular and molecular life sciences : CMLS,* 2015, vol. 72 (1), 87-99
- **BRENNECKE J ; STARK A ; RUSSELL RB ; COHEN SM.** Principles of microRNA-target recognition. *PLoS Biol.,* 2005, vol. 3 (3
- **CALIN GA ; CROCE CM.** MicroRNA signatures in human cancers. *Nat Rev Cancer.,* 2006, vol. 6 (11), 857-66
- **ESQUELA-KERSCHER A ; SLACK FJ.** Oncomirs - microRNAs with a role in cancer. *Nat Rev Cancer.,* 2006, vol. 6 (4), 259-69
- **WIEMER EA.** The role of microRNAs in cancer: no small matter. *Eur J Cancer.,* 2007, vol. 43 (10), 1529-44
- **ESTELLER M.** Non-coding RNAs in human disease. *Nature reviews Genetics,* 2011, vol. 12 (12), 861-874

- **DE GUIRE V ; ROBITAILLE R ; TETREAULT N ; GUERIN R ; MENARD C ; BAMBACE N ; SAPIEHA P.** Circulating miRNAs as sensitive and specific biomarkers for the diagnosis and monitoring of human diseases: promises and challenges. *Clinical biochemistry,* 2013, vol. 46 (10-11), 846-860
- **BRODERICK JA ; ZAMORE PD.** MicroRNA therapeutics. *Gene therapy,* 2011, vol. 18 (12), 1 104-1 1 10
- **MACK GS.** MicroRNA gets down to business. *Nature biotechnology,* 2007, vol. 25 (6), 631-638
- **LODYGIN D ; TARASOV V ; EPANCHINTSEV A ; BERKING C ; KNYAZEVA T ; KÖRNER H ; KNYAZEV P ; DIEBOLD J ; HERMEKING H.** Inactivation of miR-34a by aberrant CpG methylation in multiple types of cancer. *Cell Cycle.,* 2008, vol. 7 (16), 2591-600
- **GALLARDO E ; NAVARRO A ; VIÑOLAS N ; MARRADES RM ; DIAZ T ; GEL B ; QUERA A ; BANDRES E ; GARCIA-FONCILLAS J ; RAMIREZ J.** miR-34a as a prognostic marker of relapse in surgically resected non-small-cell lung cancer. *Carcinogenesis,* 2009, vol. 30 (11), 1903-9
- **CHIM CS ; WONG KY ; QI Y ; LOONG F ; LAM WL ; WONG LG ; JIN DY ; COSTELLO JF ; LIANG R.** Epigenetic inactivation of the miR-34a in hematological malignancies. *Carcinogenesis.,* 2010, vol. 31 (4), 745-50
- **HERMEKING H.** The miR-34 family in cancer and apoptosis. *Cell Death Differ.,* 2010, vol. 17 (2), 193-9
- **JI Q ; HAO X ; ZHANG M ; TANG W ; YANG M ; LI L ; XIANG D ; DESANO JT ; BOMMER GT ; FAN D.** MicroRNA miR-34 inhibits human pancreatic cancer tumor-initiating cells. *PLoS One,* 2009, vol. 4 (8), e6816

- **LIU C ; KELNAR K ; LIU B ; CHEN X ; CAL-HOUN-DAVIS T ; LI H ; PATRAWALA L ; YAN H ; JETER C ; HONORIO S.** The microRNA miR-34a inhibits prostate cancer stem cells and metastasis by directly repressing CD44. *Nat Med.,* 2011, vol. 17 (2), 211-5
- **BADER AG.** miR-34 - a microRNA replacement therapy is headed to the clinic. *Front Genet.,* 2012, vol. 3, 120
- **TRANG P ; WIGGINS JF ; DAIGE CL ; CHO C ; OMOTOLA M ; BROWN D ; WEIDHAAS JB ; BADER AG ; SLACK FJ.** Systemic delivery of tumor suppressor microRNA mimics using a neutral lipid emulsion inhibits lung tumors in mice. *MolTher.,* 2011, vol. 19 (6), 1116-22
- **WIGGINS JF ; RUFFINO L ; KELNAR K ; OMOTOLA M ; PATRAWALA L ; BROWN D ; BADER AG.** Development of a lung cancer therapeutic based on the tumor suppressor microRNA-34. *Cancer Res.,* 2010, vol. 70 (14), 5923-30
- **CRAIG VJ ; TZANKOV A ; FLORI M ; SCHMID CA ; BADER AG ; MÜLLER A.** Systemic microRNA-34a delivery induces apoptosis and abrogates growth of diffuse large B-cell lymphoma in vivo. *Leukemia,* 2012, vol. 26 (11), 2421-4
- **CHANG TC ; WENTZEL EA ; KENT OA ; RAMACHANDRAN K ; MULLENDORE M ; LEE KH ; FELDMANN G ; YAMAKUCHI M ; FERLITO M ; LOWENSTEIN CJ.** Transactivation of miR34a by p53 broadly influences gene expression and promotes apoptosis. *Mol Cell,* 2007, vol. 26, 745-752
- **RAVER-SHAPIRA N ; MARCIANO E ; MEIRI E ; SPECTOR Y ; ROSENFELD N ; MOSKOVITS N ; BENTWICH Z ; OREN M.** Transcriptional activation of miR34a contributes to p53-mediated apoptosis. *Mol Cell,* 2007, vol. 26, 731-743
- **CORNEY DC ; FLESKEN-NIKITIN A ; GODWIN AK ; WANG W ; NIKITIN AY.** MicroRNA-34b and MicroRNA-34c are targets of p53 and cooperate in control of cell proliferation and adhesion-independent growth. *Cancer Res.,* 2007, vol. 67 (18), 8433-8
- **HERMEKING H.** The mir 34 family in cancer and apoptosis. *Cell Death Differ,* 2010, vol. 17, 193-199
- **LI L ; YUAN L ; LUO J ; GAO J ; GUO J ; XIE X.** Mir 34a inhibits proliferation and migration of breast cancer through down regulation of bcl 2 and sirt1. *Clin Exp Med,* 2013, vol. 13, 109-117
- **LIN Q ; MAO Y ; SONG Y ; HUANG D.** MicroRNA-34a induces apoptosis in PC12 cells by reducing B-cell lymphoma 2 and sirtuin-1 expression. *Mol Med Rep,* 2015, vol. 12 (4), 5709-14
- **MATSUSHITA N ; TAKAMI Y ; KIMURA M ; TACHIIRI S ; ISHIAI M ; NAKAYAMA T ; TAKATA M.** Role of nad dependent deacetylases sirt1 and sirt2 in radiation and cisplatin induced cell death in vertebrate cells. *Genes Cells,* 2005, vol. 10, 321-332
- **CASTRO RE ; FERREIRA DM ; AFONSO MB ; BORRALHO PM ; MACHADO MV ; CORTEZ PINTO H ; RODRIGUES CM.** Mir 34a/sirt1/p53 is suppressed by ursodeoxycholic acid in the rat liver and activated by disease severity in human non alcoholic fatty liver disease. *J Hepatol,* 2013, vol. 58, 119-125
- **VARKONYI-GASIC E ; GOULD N ; SANDANAYAKA M ; SUTHERLAND P ; MACDIARMID RM.** Characterisation of microRNAs from apple (Malus domestica 'Royal Gala') vascular tissue and phloem sap. *BMC Plant Biol.,* 2010, vol. 10, 159
- **PULIDO A ; LAUFS P.** Co-ordination of developmental processes by small RNAs during leaf development. *J Exp Bot.,* 2010, vol. 61 (5), 1277-91
- **SUNKAR R ; LI YF ; JAGADEESWARAN G.** Functions of microRNAs in plant stress responses. *Trends Plant Sci.,* 2012, vol. 17 (4), 196-203
- **ZHANG L ; HOU D ; CHEN X ; LI D ; ZHU L ; ZHANG Y ; LI J ; BIAN Z ; LIANG X ; CAI X.** Exogenous plant MIR168a specifically targets mammalian LDLRAP1: evidence of cross-kingdom regulation by microRNA. *Cell Res.,* 2012, vol. 22 (1), 107-26
- **DICKINSON B ; ZHANG Y ; PETRICK JS ; HECK G ; IVASHUTA S ; MARSHALL WS.** Lack of detectable oral bioavailability of plant microRNAs after feeding in mice. *Nat Biotechnol.,* 2013, vol. 31 (11), 965-7
- **SNOW JW ; HALE AE ; ISAACS SK ; BAGGISH AL ; CHAN SY.** Ineffective delivery of diet-derived microRNAs to recipient animal organisms. *RNA Biol.,* vol. 10 (7), 1107-16
- **LIANG G ; ZHU Y ; SUN B ; SHAO Y ; JING A ; WANG J ; XIAO Z.** Assessing the survival of exogenous plant microRNA in mice. *Food SciNutr.,* 2014, vol. 2 (4), 380-8
- **CHIN AR ; FONG MY ; SOMLO G ; WU J ; SWIDERSKI P ; WU X ; WANG SE.** Cross-kingdom inhibition of breast cancer growth by plant miR159. *Cell Res.,* 2016, vol. 26 (2), 217-28
- **YANIK H ; TURKTAS M ; DUNDAR E ; HERNANDEZ P ; DORADO G ; UNVER T.** Genome-wide identification of alternate bearing-associated microRNAs (miRNAs) in olive (Olea europaea L.). *BMC Plant Biol.,* 2013, vol. 13, 10
- **PANTALEO V ; SZITTYA G ; MOXON S ; MIOZZI L ; MOULTON V ; DALMAY T ; BURGYAN.** Identification of grapevine microRNAs and their targets using high-throughput sequencing and degradome analysis. *J. Plant J.,* 2010, vol. 62 (6), 960-76
- **ALLEN E ; XIE Z ; GUSTAFSON AM ; SUNG GH ; SPATAFORA JW ; CARRINGTON JC.** Evolution of microRNA genes by inverted duplication of target gene sequences in Arabidopsis thaliana. *Nat Genet.,* 2004, vol. 36 (12), 1282-90

- **DONAIRE L ; PEDROLA L ; ROSA RDE L ; LLAVE C.** High-throughput sequencing of RNA silencing-associated small RNAs in olive (Olea europaea L.). *PLoS One,* 2011, vol. 6 (11), e27916
- **JOHN B ; ENRIGHT AJ ; ARAVIN A ; TUSCHL T ; SANDER C ; MARKS DS.** Human MicroRNA targets. *PLoS Biol.,* 2004, vol. 2 (11
- **LEWIS BP ; BURGE CB ; BARTEL DP.** Conserved seed pairing, often flanked by adenosines, indicates that thousands of human genes are microRNA targets. *Cell.,* 2005, vol. 120 (1), 15-20
- **KERTESZ M ; IOVINO N ; UNNERSTALL U ; GAUL U ; SEGAL E.** The role of site accessibility in microRNA target recognition. *Nature genetics,* 2007, vol. 39 (10), 1278-84
- **KREK A ; GRÜN D ; POY MN ; WOLF R ; ROSEN-BERG L ; EPSTEIN EJ ; MACMENAMIN P ; DA PIE-DADE I ; GUNSALUS KC ; STOFLEL M.** Combinatorial microRNA target predictions. *Nat Genet.,* 2005, vol. 37 (5), 495-500
- **CORONNELLO C ; BENOS PV.** ComiR: Combinatorial microRNA target prediction tool. *Nucleic Acids Res.,* 2013, vol. 41, W159-64
- **PIRRÒ S ; ZANELLA L ; KENZO M ; MONTESANO C ; MINUTOLO A ; POTESTÀ M ; SOBZE MS ; CANINI A ; CIRILLI M ; MULEO R.** MicroRNA from Moringa oleifera: Identification by High Throughput Sequencing and Their Potential Contribution to Plant Medicinal Value. *PLoS One,* 2016, vol. 11 (3), e0149495
- **SHAHZAD U ; KHAN M ; JASKANI M ; KHAN I ; KORBAN S.** Genetic diversity and population structure of Moringa oleifera. *Conservation Genetics.,* 2013, vol. 14 (6), 1161-1172
- **ANWAR F ; LATIF S ; ASHRAF M ; GILANI AH.** Moringa oleifera: a food plant with multiple medicinal uses. *Phytother Res.,* 2007, vol. 21 (1), 17-25
- **ABDULL RAZIS AF ; IBRAHIM MD ; KNTAYYA SB.** Health benefits or Moringa oleifera. *Asian Pac J Cancer Prev.,* 2014, vol. 15 (20), 8571-8576
- **GISMONDI A ; CANUTI L ; IMPEI S ; DI MARCO G ; KENZO M ; COLIZZI V.** Antioxidant extracts of African medicinal plants induce cell cycle arrest and differentiation in B16F10 melanoma cells. *Int J Oncol.,* 2013, vol. 43 (3), 956-964
- **JUNG IL.** Soluble extract from Moringa oleifera leaves with a new anticancer activity. *PLoS One,* 2014, vol. 9 (4), e95492
- **CHRUPEK M ; SIIPI H ; MARTINELLI L.** Bio-objects as ''boundary crawlers:'' the case of microRNAs. *Croat Med J.,* 2012, vol. 53 (3), 285-8
- **WANG B.** Base Composition Characteristics of Mammalian miRNAs. *J Nucleic Acids. 2013,* 2013, 951570
- **VERGOULIS T ; VLACHOS IS ; ALEXIOU P ; GEORGAKILAS G ; MARAGKAKIS M ; RECZKO M ; GERANGELOS S ; KOZIRIS N ; DALAMAGAS T ; HATZIGEORGIOU AG.** TarBase 6.0: capturing the exponential growth of miRNA targets with experimental support. *Nucleic acids research,* 2012, vol. 40, D222-9
- **BUSTIN SA ; BENES V ; GARSON JA ; HELLE-MANS J ; HUGGETT J ; KUBISTA M ; MUELLER R ; NOLAN T ; PFAFFL MW ; SHIPLEY GL.** The MIQE guidelines: minimum information for publication of quantitative real-time PCR experiments. *Clin Chem.,* 2009, vol. 55 (4), 611-22
- **KOZOMARA A ; GRIFFITHS-JONES S.** miRBase: integrating microRNA annotation and deep-sequencing data. *Nucleic acids research,* 2011, vol. 39, D152-7
- **CRAIG VJ ; TZANKOV A ; FLORI M ; SCHMID CA ; BADER AG ; MÜLLER A.** Systemic microRNA-34a delivery induces apoptosis and abrogates growth of diffuse large B-cell lymphoma in vivo. *Leukemia,* 2012, vol. 26 (13), 2421-4
- **LI XJ ; REN ZJ2 ; TANG JH1.** MicroRNA-34a: a potential therapeutic target in human cancer. *Cell Death Dis.,* 2014, vol. 5, e1327